(19) Europäisches Patentamt
European Patent Office
Office européen des brevets

(11) EP 0 667 863 B1

(12) **EUROPÄISCHE PATENTSCHRIFT**

(45) Veröffentlichungstag und Bekanntmachung des Hinweises auf die Patenterteilung:
**03.06.1998 Patentblatt 1998/23**

(21) Anmeldenummer: **93923547.9**

(22) Anmeldetag: **25.10.1993**

(51) Int Cl.[6]: **C07D 235/10**, C07D 491/056, C07D 405/12, A01N 43/52, A01N 43/90 // (C07D491/056, 317:00, 235:00), (C07D491/056, 319:00, 235:00), (C07D491/056, 321:00, 235:00)

(86) Internationale Anmeldenummer:
**PCT/EP93/02948**

(87) Internationale Veröffentlichungsnummer:
**WO 94/11351 (26.05.1994 Gazette 1994/12)**

(54) **2-PERHALOGENALKYL-SUBSTITUIERTE BENZIMIDAZOLE, IHRE HERSTELLUNG UND IHRE VERWENDUNG ALS SCHÄDLINGSBEKÄMPFUNGSMITTEL**

2-PERHALOGENALKYL-SUBSTITUTED BENZIMIDAZOLES, THEIR PREPARATION AND THEIR USE AS PESTICIDES

BENZIMIDAZOLES 2-PERHALOGENOALKYL-SUBSTITUES, LEUR FABRICATION ET LEUR UTILISATION COMME PESTICIDES

(84) Benannte Vertragsstaaten:
**AT BE CH DE DK ES FR GB IE IT LI NL PT SE**

(30) Priorität: **06.11.1992 DE 4237548**

(43) Veröffentlichungstag der Anmeldung:
**23.08.1995 Patentblatt 1995/34**

(73) Patentinhaber: **BAYER AG**
**51368 Leverkusen (DE)**

(72) Erfinder:
- **LUNKENHEIMER, Winfried**
  **D-42115 Wuppertal (DE)**
- **BAASNER, Bernd**
  **D-51467 Bergisch Gladbach (DE)**
- **LIEB, Folker**
  **D-51375 Leverkusen (DE)**
- **ERDELEN, Christoph**
  **D-42799 Leichlingen (DE)**
- **HARTWIG, Jürgen**
  **D-42799 Leichlingen (DE)**
- **WACHENDORFF-NEUMANN, Ulrike**
  **D-40789 Monheim (DE)**
- **STENDEL, Wilhelm**
  **D-42113 Wuppertal (DE)**
- **GÖRGENS, Ulrich**
  **D-40882 Ratingen (DE)**

(56) Entgegenhaltungen:
**EP-A- 0 251 013** **EP-A- 0 487 286**
**DE-A- 1 545 865** **DE-A- 1 670 786**
**DE-A- 2 509 346**

- **CHEMICAL ABSTRACTS, vol. 66, no. 7, 13. Februar 1967, Columbus, Ohio, US; abstract no. 28771p, 'Herbicidal benzimidazoles' Seite 2743 ;Spalte 2 ;**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 667 863 B1

## Beschreibung

Die Erfindung betrifft neue substituierte Benzimidazole, mehrere Verfahren zu ihrer Herstellung und ihre Verwendung als Schädlingsbekämpfungmittel.

Es ist bekannt, daß bestimmte Phosphorsäureester oder Carbamate wie beispielsweise die Verbindung O,S-Dimethyl-thiolo-phosphorsäureamid oder die Verbindung N-Methyl-O-(2-isopropoxyphenyl)-carbamat insektizide Eigenschaften besitzen (vergl. z.B. DE 12 10 835 bzw. DE 11 08 202).

1-Cyanomethyl-2-trifluormethyl-5,6-dichlorbenzimidazol und seine insektizide Wirkung war bekannt aus GB-P 1 213 796. Insektizide und herbizide Wirkung von z.B. 2-Trifluormethyl-5,6-dichlor-benzimidazol, 1-Allyl-2-Trifluormethyl-5,6-dichlor-benzimidazol war bekannt aus Chemical Abstracts Vol.66 (1967): 28771p und DE-P 1 670 786.

Die Wirkungshöhe bzw. Wirkungsdauer dieser vorbekannten Verbindungen ist jedoch, insbesondere bei bestimmten Insekten oder bei niedrigen Anwendungskonzentrationen, nicht für alle Anwendungsgebiete völlig zufriedenstellend.

Es wurden neue substituierte Benzimidazole der allgemeinen Formel (I) gefunden

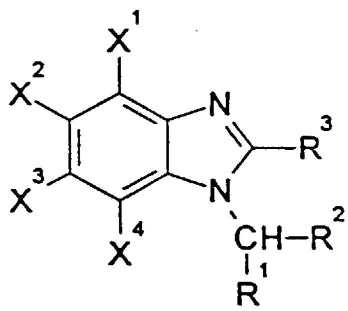

(I)

in welcher

$R^1$ für Wasserstoff, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxyalkyl, Alkoxyalkoxy, Alkanoyl, Alkoxycarbonyl oder Alkoximinoalkyl mit jeweils 1 bis 6 Kohlenstoffatomen in den einzelnen Alkylteilen, gegebenenfalls einfach oder mehrfach, gleich oder verschieden durch Halogen und/oder geradkettiges oder verzweigtes Alkyl mit 1 bis 6 Kohlenstoffatomen und/oder geradkettiges oder verzweigtes Halogenalkyl mit 1 bis 6 Kohlenstoffatomen und 1 bis 13 gleichen oder verschiedenen Halogenatomen substituiertes, zweifach verknüpftes Dioxyalkylen mit 1 bis 5 Kohlenstoffatomen oder gegebenenfalls einfach oder mehrfach, gleich oder verschieden durch Halogen und/oder geradkettiges oderverzweigtes Alkyl mit 1 bis 6 Kohlenstoffatomen und/oder geradkettiges oder verzweigtes Halogenalkyl mit 1 bis 6 Kohlenstoffatomen und 1 bis 13 gleichen oder verschiedenen Halogenatomen substituiertes Phenyl,

$R^2$ für Hydroxy, Cyano, Alkoxy mit 1 bis 8 Kohlenstoffatomen oder für gegebenenfalls einfach oder zweifach, gleich oder verschieden substituiertes Amino steht, wobei als Substituenten infrage kommen:
geradkettiges oder verzweigtes Alkyl mit 1 bis 8 Kohlenstoffatomen, geradkettiges oder verzweigtes Alkenyl mit 2 bis 8 Kohlenstoffatomen, Cycloalkyl mit 3 bis 8 Kohlenstoffatomen, Alkoxycarbonyl, Alkylthio-carbonyl, Alkoxy-thiocarbonyl oder Alkylthio-thiocarbonyl mit jeweils 1 bis 8 Kohlenstoffatomen in den einzelnen geradkettigen oder verzweigten Alkylteilen, zweifach verknüpftes, ringgeschlossenes Alkandiyloxycarbonyl mit 2 bis 6 Kohlenstoffatomen im Alkandiylteil oder jeweils gegebenenfalls einfach oder mehrfach, gleich oder verschieden substituiertes Phenyl oder Phenylalkyl mit 1 bis 6 Kohlenstoffatomen im geradkettigen oder verzweigten Alkylteil, wobei als Substituenten die bei $R^1$ genannten infrage kommen,

$R^3$ für geradkettiges oder verzweigtes Perhalogenalkyl mit 1 bis 8 Kohlenstoffatomen und 1 bis 17 gleichen oder verschiedenen Halogenatomen steht und

$X^1$,$X^2$,$X^3$ und $X^4$ unabhängig voneinander jeweils für Wasserstoff, Fluor, Chlor, Brom, Iod, Nitro oder für gegebenenfalls einfach oder mehrfach, gleich oder verschieden substituiertes Phenoxy stehen, wobei als Substituenten die bei $R^1$ genannten infrage kommen wobei jedoch mindestens einer der Substituenten $X^1$, $x^2$, $X^3$ oder $X^4$ verschieden von Wasserstoff ist und

wobei die Verbindung 1-Cyanomethyl-2-trifluormethyl-5,6-dichlorbenzimidazol ausgenommen ist.

Besonders bevorzugt sind Verbindungen der Formel (I), bei welchen

$R^1$ für Wasserstoff oder für geradkettiges oder verzweigtes Alkyl mit 1 bis 6 Kohlenstoffatomen steht,

$R^2$ für Hydroxy, Cyano, Alkoxy mit 1 bis 6 Kohlenstoffatomen oder für gegebenenfalls einfach oder zweifach, gleich oder verschieden substituiertes Amino steht, wobei als Substituenten infrage kommen:
geradkettiges oder verzweigtes Alkyl mit 1 bis 6 Kohlenstoffatomen, geradkettiges oder verzweigtes Alkenyl mit 2 bis 6 Kohlenstoffatomen, Cycloalkyl mit 3 bis 7 Kohlenstoffatomen, Alkoxycarbonyl, Alkylthio-carbonyl, Alkoxy-thiocarbonyl oder Alkylthio-thiocarbonyl mit jeweils 1 bis 6 Kohlenstoffatomen in den einzelnen geradkettigen oder verzweigten Alkylteilen.

$R^3$ für geradkettiges oder verzweigtes Perhalogenalkyl mit 1 bis 6 Kohlenstoffatomen und 1 bis 13 gleichen oder verschiedenen Halogenatomen steht und

$X^1$,$X^2$,$X^3$ und $X^4$ unabhängig voneinander jeweils für Wasserstoff, Fluor, Chlor, Brom, Iod, Nitro oder für gegebenenfalls einfach bis fünffach, gleich oder verschieden substituiertes Phenoxy stehen, wobei als Substituenten die folgenden infrage kommen
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxyalkyl, Alkoxyalkoxy, Alkanoyl, Alkoxycarbonyl oder Alkoximinoalkyl mit jeweils 1 bis 4 Kohlenstoffatomen in den einzelnen Alkylteilen, gegebenenfalls einfach bis sechsfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, zweifach verknüpftes Dioxyalkylen mit 1 bis 4 Kohlenstoffatomen oder gegebenenfalls einfach bis fünffach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes Phenyl,
wobei jedoch mindestens einer der Substituenten $X^1$,$X^2$, $X^3$ oder $X^4$ verschieden von Wasserstoff ist und

wobei die Verbindung 1-Cyanomethyl-2-trifluormethyl-5,6-dichlorbenzimidazol ausgenommen ist.

Ganz besonders bevorzugt sind Verbindungen der Formel (I), bei welchen

$R^1$ für Wasserstoff, für geradkettiges oder verzweigtes Alkyl mit 1 bis 4 Kohlenstoffatomen

$R^2$ für Hydroxy, Cyano, Alkoxy mit 1 bis 6 Kohlenstoffatomen oder für gegebenenfalls einfach oder zweifach, gleich oder verschieden substituiertes Amino steht, wobei als Substituenten infrage kommen:
geradkettiges oder verzweigtes Alkyl mit 1 bis 4 Kohlenstoffatomen, geradkettiges oder verzweigtes Alkenyl mit 2 bis 4 Kohlenstoffatomen, Cycloalkyl mit 3 bis 6 Kohlenstoffatomen, Alkoxycarbonyl, Alkylthio-carbonyl, Alkoxy-thiocarbonyl oder Alkylthio-thiocarbonyl mit jeweils 1 bis 4 Kohlenstoffatomen in den einzelnen geradkettigen oder verzweigten Alkylteilen,

$R^3$ für geradkettiges oder verzweigtes Perhalogenalkyl mit 1 bis 4 Kohlenstoffatomen und 1 bis 9

gleichen oder verschiedenen Halogenatomen steht und

$X^1$, $X^2$, $X^3$ und $X^4$ unabhängig voneinander jeweils für Wasserstoff, Chlor, Brom, Nitro oder für gegebenenfalls im Phenylteil einfach bis dreifach, gleich oder verschieden substituiertes Phenyloxy stehen, wobei als Phenylsubstituenten jeweils die folgenden infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweilsl bis 3 Kohlenstoffatomen und 1 bis 7 gleichen oder verschiedenen Halogenatomen,

wobei jedoch mindestens einer der Substituenten $X^1$, $X^2$, $X^3$ oder $X^4$ verschieden von Wasserstoff ist und wobei die Verbindung 1-Cyanomethyl-2-trifluormethyl-5,6-dichlorbenzimidazol ausgenommen ist.

Im einzelnen seien außer den bei den Herstellungsbeispielen genannten Verbindungen die folgenden substituierten Benzimidazole der allgemeinen Formel (I) genannt:

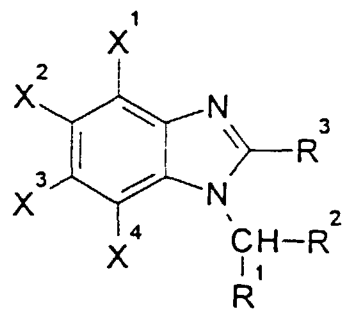

(I)

| $X^1$ | $X^2$ | $X^3$ | $X^4$ | $R^1$ | $R^2$ | $R^3$ |
|---|---|---|---|---|---|---|
| Br | H | Cl | H | H | 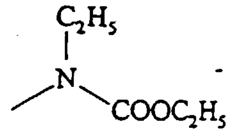 | $CF_3$ |
| Br | H | Br | H | H | 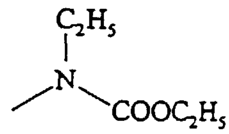 | $CF_3$ |
| Br | H | $NO_2$ | H | H | 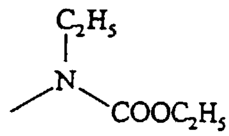 | $CF_3$ |
| Cl | H | Cl | H | H | 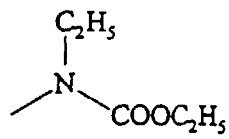 | $CF_3$ |
| Cl | H | Br | H | H | 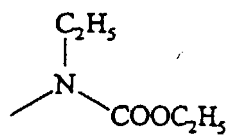 | $CF_3$ |
| H | H | $NO_2$ | H | H | 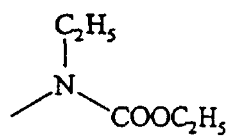 | $CF_3$ |
| Br | H | $C_6H_5O$ | H | H | 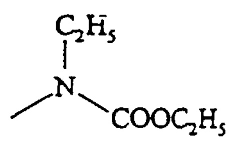 | $CF_3$ |
| Cl | H | 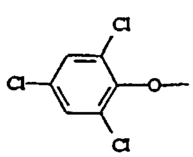 | H | H | 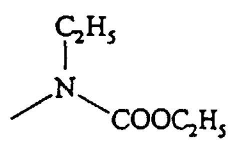 | $CF_3$ |

| $X^1$ | $X^2$ | $X^3$ | $X^4$ | $R^1$ | $R^2$ | $R^3$ |
|---|---|---|---|---|---|---|
| Cl | H | 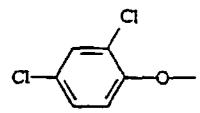 | H | H | 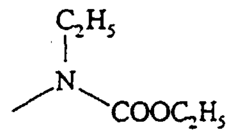 | $CF_3$ |
| Br | H | Cl | H | H | $-O-C_2H_5$ | $CF_3$ |
| Br | H | Br | H | H | $-O-C_2H_5$ | $CF_3$ |
| Br | H | $NO_2$ | H | H | $-O-C_2H_5$ | $CF_3$ |
| Cl | H | Br | H | H | $-O-C_2H_5$ | $CF_3$ |
| H | H | $NO_2$ | H | H | $-O-C_2H_5$ | $CF_3$ |
| Br | H | $C_6H_5O$ | H | H | $-O-C_2H_5$ | $CF_3$ |
| Cl | H | 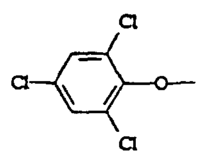 | H | H | $-O-C_2H_5$ | $CF_3$ |
| Cl | H | 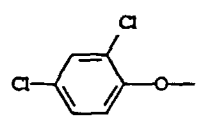 | H | H | $-O-C_2H_5$ | $CF_3$ |
| Br | H | Cl | H | H | 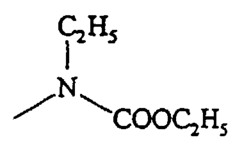 | $C_2F_5$ |
| Br | H | Br | H | H | 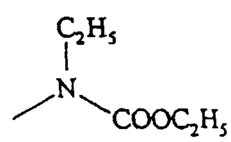 | $C_2F_5$ |
| H | Cl | Cl | H | H | 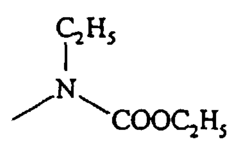 | $C_2F_5$ |

| $X^1$ | $X^2$ | $X^3$ | $X^4$ | $R^1$ | $R^2$ | $R^3$ |
|---|---|---|---|---|---|---|
| Br | H | $NO_2$ | H | H | 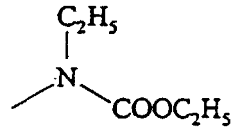 | $C_2F_5$ |
| Cl | H | Cl | H | H | 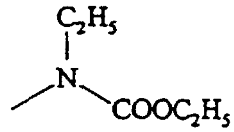 | $C_2F_5$ |
| Cl | H | Br | H | H | 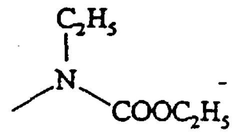 | $C_2F_5$ |
| H | H | $NO_2$ | H | H | 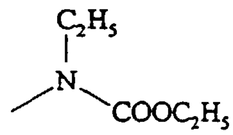 | $C_2F_5$ |
| Br | H | $C_6H_5O$ | H | H | 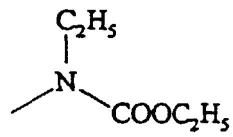 | $C_2F_5$ |
| Cl | H | 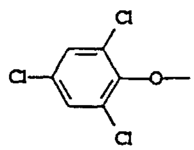 | H | H | 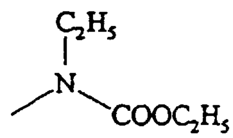 | $C_2F_5$ |
| Cl | H | 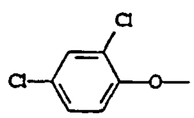 | H | H | 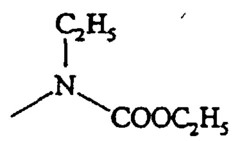 | $C_2F_5$ |
| Br | H | Cl | H | H | $-O-C_2H_5$ | $C_2F_5$ |
| Br | H | Br | H | H | $-O-C_2H_5$ | $C_2F_5$ |
| H | Cl | Cl | H | H | $-O-C_2H_5$ | $C_2F_5$ |
| Br | H | $NO_2$ | H | H | $-O-C_2H_5$ | $C_2F_5$ |
| Cl | H | Cl | H | H | $-O-C_2H_5$ | $C_2F_5$ |
| Cl | H | Br | H | H | $-O-C_2H_5$ | $C_2F_5$ |
| H | H | $NO_2$ | H | H | $-O-C_2H_5$ | $C_2F_5$ |
| Br | H | $C_6H_5O$ | H | H | $-O-C_2H_5$ | $C_2F_5$ |

| $X^1$ | $X^2$ | $X^3$ | $X^4$ | $R^1$ | $R^2$ | $R^3$ |
|---|---|---|---|---|---|---|
| Cl | H | 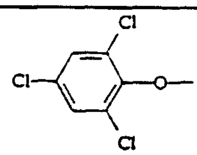 | H | H | $-O-C_2H_5$ | $C_2F_5$ |
| Cl | H | 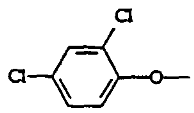 | H | H | $-O-C_2H_5$ | $C_2F_5$ |
| Br | H | Cl | H | H | 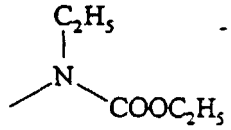 | $n-C_3F_7$ |
| Br | H | Br | H | H | 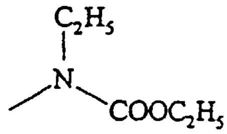 | $n-C_3F_7$ |
| H | Cl | Cl | H | H | 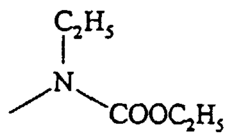 | $n-C_3F_7$ |
| Br | H | $NO_2$ | H | H | 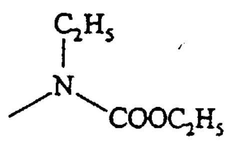 | $n-C_3F_7$ |
| Cl | H | Cl | H | H | 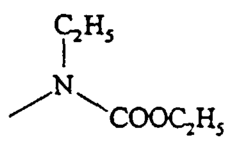 | $n-C_3F_7$ |

| $X^1$ | $X^2$ | $X^3$ | $X^4$ | $R^1$ | $R^2$ | $R^3$ |
|---|---|---|---|---|---|---|
| Cl | H | Br | H | H | 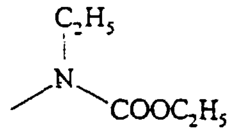 | $n\text{-}C_3F_7$ |
| H | H | $NO_2$ | H | H | 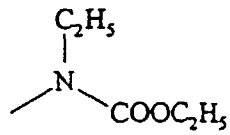 | $n\text{-}C_3F_7$ |
| Br | H | $C_6H_5O$ | H | H | 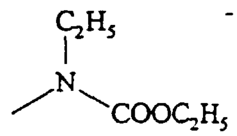 | $n\text{-}C_3F_7$ |
| Cl | H | 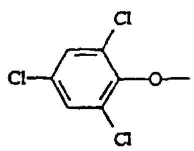 | H | H | 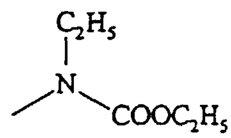 | $n\text{-}C_3F_7$ |
| Cl | H | 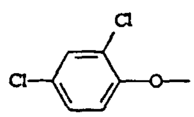 | H | H | 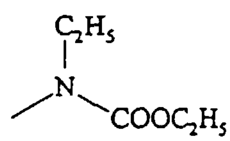 | $n\text{-}C_3F_7$ |
| Br | H | Cl | H | H | $-O-C_2H_5$ | $n\text{-}C_3F_7$ |
| Br | H | Br | H | H | $-O-C_2H_5$ | $n\text{-}C_3F_7$ |
| H | Cl | Cl | H | H | $-O-C_2H_5$ | $n\text{-}C_3F_7$ |
| Br | H | $NO_2$ | H | H | $-O-C_2H_5$ | $n\text{-}C_3F_7$ |
| Cl | H | Cl | H | H | $-O-C_2H_5$ | $n\text{-}C_3F_7$ |
| Cl | H | Br | H | H | $-O-C_2H_5$ | $n\text{-}C_3F_7$ |
| H | H | $NO_2$ | H | H | $-O-C_2H_5$ | $n\text{-}C_3F_7$ |
| Br | H | $C_6H_5O$ | H | H | $-O-C_2H_5$ | $n\text{-}C_3F_7$ |

| $X^1$ | $X^2$ | $X^3$ | $X^4$ | $R^1$ | $R^2$ | $R^3$ |
|---|---|---|---|---|---|---|
| Cl | H | 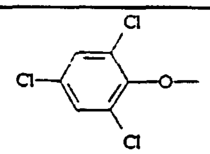 | H | H | $-O-C_2H_5$ | $n-C_3F_7$ |
| Cl | H | 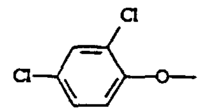 | H | H | $-O-C_2H_5$ | $n-C_3F_7$ |
| Cl | H | 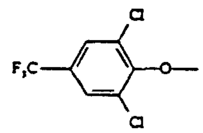 | H | H | $-O-C_2H_5$ | $CF_3$ |
| Cl | H | 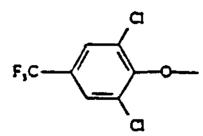 | H | H | $-O-C_2H_5$ | $C_2F_5$ |
| Cl | H | 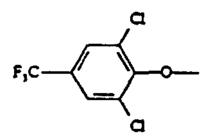 | H | H | $-O-C_2H_5$ | $n-C_3F_7$ |
| Cl | H | 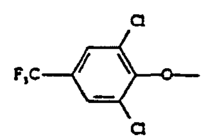 | H | H | 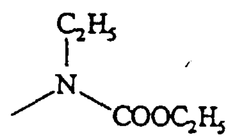 | $CF_3$ |
| Cl | H | 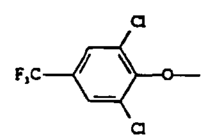 | H | H | 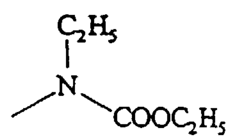 | $C_2F_5$ |
| Cl | H | 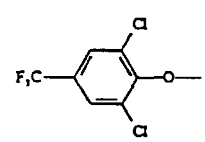 | H | H | 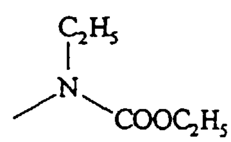 | $n-C_3F_7$ |

Die Verbindungen der Formel (I) können gegebenenfalls in Abhängigkeit von der Art und Anzahl der Substituenten als geometrische und/oder optische Isomere bzw. Regioisomere oder deren Isomerengemische in unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Benzimidazole der allgemeinen Formel (I),

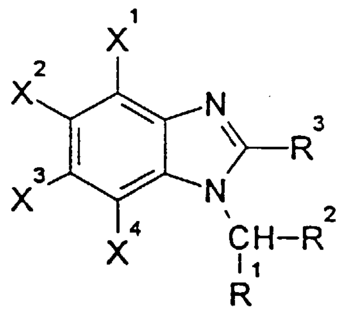

(I)

in welcher die Substituenten die oben angegebene Bedeutung besitzen wobei die Verbindung 1-Cyanomethyl-2-trifluormethyl-5,6-dichlorbenzimidazol ausgenommen ist,
erhält, wenn man 1H-Benzimidazole der Formel (II),

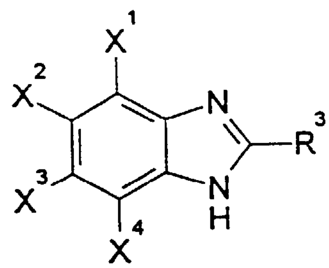

in welcher

$R^3$,$X^1$, $X^2$, $X^3$ und $X^4$ die oben angegebene Bedeutung haben, mit Verbindungen der Formel (III),

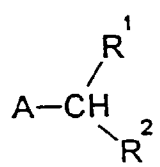

(III)

in welcher
A für eine geeignete Abgangsgruppe steht,
$R^1$ die oben angegebene Bedeutung hat und
$R^2$ die oben angegebene Bedeutung hat gegebenenfalls in Gegenwart eines Verdünnungsmittels und

gegebenenfalls in Gegenwart eines Reaktionshilfsmittels umsetzt.

Schließlich wurde gefunden, daß die neuen substituierten Benzimidazole der allgemeinen Formel (I) gute Wirksamkeit gegen Schädlinge besitzen.

Überraschenderweise zeigen die erfindungsgemäßen substituierten Benzimidazole der allgemeinen Formel (I) eine erheblich bessere insektizide Wirksamkeit im Vergleich zu aus dem Stand der Technik bekannten Verbindungen.

Verwendet man beispielsweise 5,6-Dichlor-2-trifluormethyl-benzimidazol und Chlormethylethylether als Ausgangsverbindungen, so läßt sich der Reaktionsablauf des erfindungsgemäßen Verfahrens durch das folgende Formelschema darstellen:

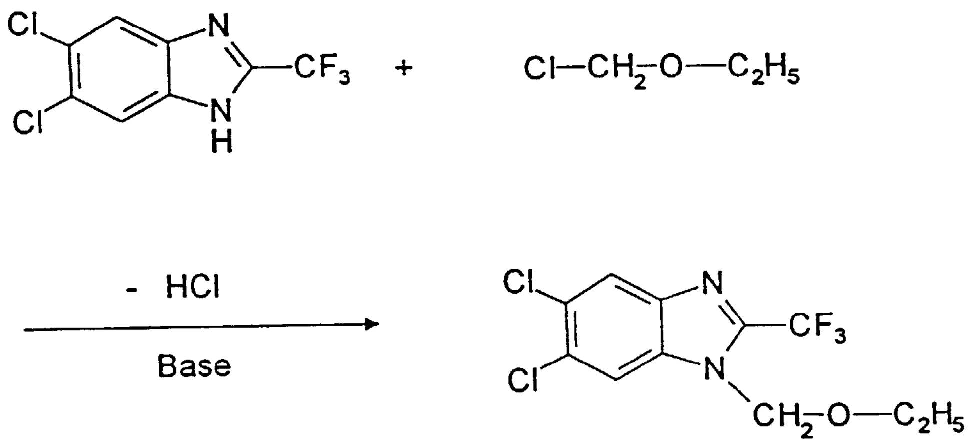


Die zur Durchführung des erfindungsgemäßen Verfahrens als Ausgangsstoffe benötigten 1H-Benzimidazole sind durch die Formel (II) allgemein definiert. In dieser Formel (II) stehen $R^3$, $X^1$, $X^2$, $X^3$ und $X^4$ vorzugsweise für diejenigen Reste, die bereits im Zusammenhang mit der Beschreibung der erfindungsgemäßen Verbindungen der Formel (I) als bevorzugt für diese Substituenten genannt wurden.

Die 1H-Benzimidazole der Formel (II) sind bekannt oder erhältlich in Analogie zu bekannten Verfahren (vergl. z. B. J. Amer. Chem. Soc. 75, 1292 [1953]; US 3.576.818)

Die zur Durchführung des erfindungsgemäßen Verfahrens weiterhin als Ausgangsprodukte erforderlichen Verbindungen sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

A steht vorzugsweise für einen bei Alkylierungsmitteln üblichen Abgangsrest, vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

A steht außerdem auch für eine Alkohol-, Alkanoyloxy- oder Alkoxygruppe, wie beispielsweise eine Hydroxy-, Acetoxy- oder Methoxygruppe, wenn mit Hilfe des erfindungsgemäßen Verfahrens Verbindungen der Formel (I), bei welchen $R^1$ verschieden von Wasserstoff ist, hergestellt werden sollen.

Die Verbindungen der Formel (III) sind bekannt oder erhältlich in Analogie zu bekannten Verfahren (vergl. z.B. DE 20 40 175; DE 21 19 518; Synthesis 1973, 703).

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutylketon; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Basen wie Pyridin oder organische Säuren, wie Ameisensäure oder Essigsäure.

Das erfindungsgemäße Verfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Lithium-diethylamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, Lithium-organische Verbindungen, wie n-Butyllithium sowie tertiäre Amine, wie Trimethylamin, Triethylamin, Tributylamin, Di-isopropyl-ethylamin, Tetramethylguanidin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

In den Fällen, wo mit Hilfe des erfindungsgemäßen Verfahrens Verbindungen der Formel (I), bei welchen $R^1$ verschieden von Wasserstoff ist, hergestellt werden sollen, kommen als Reaktionshilfsmittel auch organische oder anorganische Säuren, wie beispielsweise Schwefelsäure, Salzsäure, p-Toluolsulfonsäure, Perfluorbutansulfonsäure oder stark saure Ionenaustauscher infrage.

Das erfindungsgemäße Verfahren kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-$C_{13}$/$C_{15}$-alkylammoniumchlorid, Trimethyl-$C_{13}$/$C_{15}$-alkylammoniumbromid, Dibenzyl-dimethyl-ammoniummethylsulfat, Dimethyl-$C_{12}$/$C_{14}$-alkyl-benzylammoniumchlorid, Dimethyl-$C_{12}$/$C_{14}$-alkyl-benzylammoniumbromid. Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können bei der Durchführung des erfindungsgemäßen Verfahrens in einem größeren Bereich variiert werden. Im allgemeinen arbeitet man bei Temperaturen zwischen -70°C und +200°C, vorzugsweise bei Temperaturen zwischen 0°C und 130°C.

Das erfindungsgemäße Verfahren wird üblicherweise unter Normaldruck durchgeführt. Es ist jedoch auch möglich unter erhöhtem oder vermindertem Druck zu arbeiten.

Zur Durchführung des erfindungsgemäßen Verfahrens setzt man pro Mol an 1H-Benzimidazol der Formel (II) im allgemeinen 1,0 bis 5,0 Mol, vorzugsweise 1,0 bis 2,5 Mol an Verbindung der Formel (III) und gegebenenfalls 0,01 bis 5,0 Mol, vorzugsweise 1,0 bis 3,0 Mol an Reaktionshilfsmittel ein.

In einer besonderen Durchführungsform ist es auch möglich, die 1H-Benzimidazole der Formel (II) zunächst in einem vorgelagerten Reaktionsschritt mit Hilfe üblicher Silylierungsverfahren beispielsweise mit Hexamethyldisilazan oder Trimethylsilylchlorid, gegebenenfalls in Gegenwart eines geeigneten Katalysators, wie beispielsweise Schwefelsäure, Trifluoressigsäure, Ammoniumsulfat, Imidazol oder Saccharin bei Temperaturen zwischen -20°C und +150°C zu silylieren und die so erhältlichen 1-Trimethylsilylbenzimidazole in einer anschließenden zweiten Stufe mit Alkylierungsmitteln der Formel (II) gemäß dem erfindungsgemäßen Verfahren umzusetzen. In diesem Fall ist es von Vorteil als Katalysator zur Alkylierungsreaktion Zinntetrachlorid zuzusetzen (vergl. z.B. Chem. Heterocycl. Comp. USSR 24, 514 [1988])

Die Reaktionsdurchführung, Aufarbeitung und Isolierung der Reaktionsprodukte erfolgt nach bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen - insbesondere bei Regioisomerengemischen - mit Hilfe der Protonen-Kernresonanzspektroskopie ($^1$H-NMR).

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam.

Zu den oben erwähnten Schädlingen gehören:

Aus der Ordnung der Isopoda z.B. Oniscus asellus, Armadillidium vulgare, Porcellio scaber;
aus der Ordnung der Diplopoda z.B. Blaniulus guttulatus;
aus der Ordnung der Chilopoda z.B. Geophilus carpophagus, Scutigera spec.;
aus der Ordnung der Symphyla z.B. Scutigerella immaculata;
aus der Ordnung der Thysanura z.B. Lepisma saccharina;
aus der Ordnung der Collembola z.B. Onychiurus armatus;
aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria;
aus der Ordnung der Dermaptera z.B. Forficula auricularia;
aus der Ordnung der Isoptera z.B. Reticulitermes spp.;
aus der Ordnung der Anoplura z.B. Phylloxera vastatrix, Pemphigus spp., Pediculus humanus corporis, Haematopinus spp., Linognathus spp.;
aus der Ordnung der Mallophaga z.B. Trichodectes spp., Damalinea spp.;aus der Ordnung der Thysanoptera z. B. Hercinothrips femoralis, Thrips tabaci;
aus der Ordnung der Heteroptera z.B. Eurigaster spp., Dysdercus intermedius, Piesma quadrata, Cimex lectularius, Rhodnius prolixus, Triatoma spp.;
aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus. Nephotettix cincticeps. Lecanium corni, Saissetia oleae, Laodelphax striatellus, Nilaparvata lugens, Aonidiella aurantii, Aspidiotus hederae, Pseudococcus spp., Psylla spp.;

aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana;
aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica;
aus der Ordnung der Hymenoptera z.B. Diprion spp., Hoplocampa spp., Lasius spp., Monomorium pharaonis, Vespa spp.;
aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa;
aus der Ordnung der Siphonaptera z.B. Xenopsylla cheopis. Ceratophyllus spp.;
aus der Ordnung der Arachnida z.B. Scorpio maurus, Latrodectus mactans;
aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme, sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide Wirksamkeit aus.

Sie lassen sich mit besonders gutem Erfolg zur Bekämpfung von pflanzenschädigenden Insekten, wie beispielsweise gegen die Larven der Meerettichblattkäfer (Phaedon cochleariae) oder gegen die Raupen der Kohlschabe (Plutella maculipennis) oder gegen die Tabakknospenraupe (Heliothis virescens) ebenso wie zur Bekämpfung von pflanzenschädigenden Milben, wie beispielsweise gegen die gemeine Spinnmilbe (Tetranychus urticae) oder zur Bekämpfung von pflanzenschädigenden Nematoden, wie beispielsweise gegen die Nematodenart Globodera rostochiensis einsetzen.

Daneben lassen sich die erfindungsgemäßen Wirkstoffe auch zur Bekämpfung von Hygiene- und Vorratsschädlingen, wie beispielsweise gegen die Stubenfliege (Musca domestica) oder gegen Schabenarten, wie beispielsweise Periplaneta americana einsetzen.

Darüber hinaus lassen sich die erfindungsgemaßen Wirkstoffe mit besonders gutem Erfolg zur Bekämpfung von parasitisch lebenen Warmblüterschädlingen, wie beispielsweise gegen Räudemilben (Psoroptes ovis) einsetzen.

Darüberhinaus besitzen die erfindungsgemäßen Wirkstoffe auch eine fungizide in vitro-Wirksamkeit.

Die Wirkstoffe können in Abhängigkeit von ihren jeweiligen physikalischen und/oder chemischen Eigenschaften in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische

Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Tragerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infragen: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zinn verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden hählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetze Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gewichtsprozent Wirkstoff, vorzugsweise zwischen 0,0001 und 1 Gewichtsprozent liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise, beispielsweise durch orale Anwendung in Form von Tabletten, Kapseln, Tränken oder Granulaten, durch dermale bzw. äußerliche Anwendung in Form beispielsweise des Tauchens (Dippen), Sprühens (Sprayen), Aufgießens (pour-on oder spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die Herstellung und die Verwendung der erfindungsgemäßen Wirkstoffe geht aus den nachfolgenden Beispielen hervor.

**Herstellungsbeispiele:**

Beipiel 1:

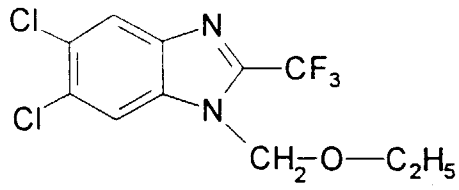

In eine Mischung von 36 g (0,12 Mol) 5,6-Dichlor-2-trifluormethyl-1H-benzimidazol, 33 g (0,24 Mol) gepulvertem Kaliumcarbonat und 300 ml Essigester tropft man bei Rückflußtemperatur eine Lösung von 14,1 g (0,15 Mol) Chlormethylethylether in 40 ml Essigester und erhitzt nach beendeter Zugabe für weitere 4 Stunden auf Siedetemperatur. Zur Aufarbeitung wird das abgekühlte Reaktionsgemisch zweimal mit jeweils 150 ml Wasser gewaschen, über Natriumsulfat getrocknet und im Vakuum eingeengt. Der Rückstand wird durch Chromatographie an Kieselgel (Laufmittel: Dichlormethan) gereinigt.

Man erhält 32, 4 g (83 % der Theorie) an 5,6-Dichlor-1-ethoxymethyl-2-trifluormethyl-benzimidazol vom Schmelzpunkt 89-92°C.

In entsprechender Weise und gemäß den allgemeinen Angaben zur Herstellung erhält man die folgenden substituierten Benzimidazole der allgemeinen Formel (I):

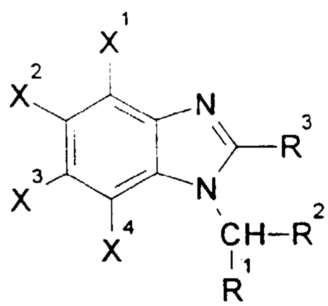

(I)

| Bsp.-Nr. | $X^1$ | $X^2$ | $X^3$ | $X^4$ | $R^1$ | $R^2$ | $R^3$ | physikalische Eigenschaften |
|---|---|---|---|---|---|---|---|---|
| 2 | H | Cl | Cl | H | H | 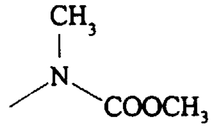 | $CF_3$ | Fp. 120-121°C |
| 3 | H | Cl | Cl | H | H | 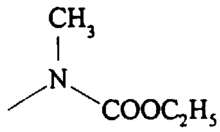 | $CF_3$ | Fp. 95-97°C |
| 4 | H | Cl | Cl | H | H | 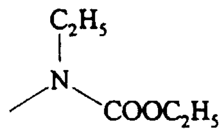 | $CF_3$ | Fp. 104-106°C |
| 5 | H | Cl | Cl | H | H | 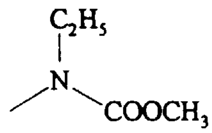 | $CF_3$ | Fp. 88-89°C |
| 6 | H | Cl | Cl | H | H | 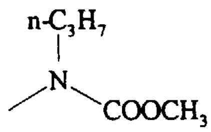 | $CF_3$ | Fp. 102-103°C |
| 7 | Cl<br>(H) | H<br>(Cl) | Cl<br>(H) | H<br>(Cl) | H | $-O-C_2H_5$ | $CF_3$ | Fp. 57-61°C<br>(87:13) |
| 8 | Cl<br>(H) | H<br>(Cl) | Cl<br>(H) | H<br>(Cl) | H | 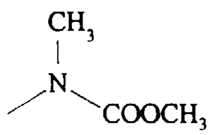 | $CF_3$ | Fp. 95-100°C<br>(92:8) |

| Bsp.-Nr. | $X^1$ | $X^2$ | $X^3$ | $X^4$ | $R^1$ | $R^2$ | $R^3$ | physikalische Eigenschaften |
|---|---|---|---|---|---|---|---|---|
| 9 | H | Cl<br>(H) | H<br>(Cl) | H | H | $-O-CH(CH_3)_2$ | $CF_3$ | $^1H$-NMR*): 5.63; 5.65; 7.35; 7.40; 7.57; 7.78 ; 7.63; 7.85 |
| 10 | H | Cl<br>(H) | H<br>(Cl) | H | H | CN | $CF_3$ | $^1H$-NMR*): 5.15; 5.18; 7.45; 7.55; 7.83; 7.90 |
| 11 | H | Cl<br>(H) | H<br>(Cl) | H | H | $-O-C_2H_5$ | $CF_3$ | $^1H$-NMR*): 5.43; 5.48; 7.28-8.01 |
| 12 | H | Cl | H | H | H | $-O-C_2H_5$ | $CF_3$ | Fp. 75°C |
| 13 | H | H | Cl | H | H | $-O-C_2H_5$ | $CF_3$ | Fp. 73°C |
| 14 | H | Cl<br>(H) | H<br>(Cl) | H | H | 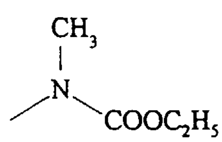 | $CF_3$ | $^1H$-NMR*): 5,88; 5.92; 7.35; 7.67; 7.92 |
| 15 | H | Cl<br>(H) | H<br>(Cl) | H | H | 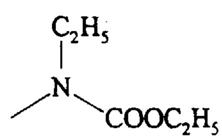 | $CF_3$ | $^1H$-NMR*): 5.88; 5.90; 7.41; 7.79; 7.81 |
| 16 | H | Cl<br>(H) | H<br>(Cl) | H | H | 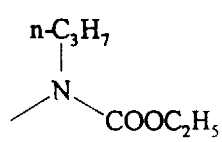 | $CF_3$ | $^1H$-NMR*): 5.89; 5.96; 7.37; 7.78; 7.78; 7.81 |
| 17 | H | Cl<br>(H) | H<br>(Cl) | H | H | OH | $CF_3$ | |
| 18 | H | $NO_2$<br>(H) | H<br>($NO_2$) | H | H | $-O-C_2H_5$ | $CF_3$ | $^1H$-NMR*): 7.79-8.78; A: 5.73 B: 5.74 |
| 19 | H | $NO_2$ | H | H | H | $-O-C_2H_5$ | $CF_3$ | Fp.: 48°C |

| Bsp.-Nr. | $X^1$ | $X^2$ | $X^3$ | $X^4$ | $R^1$ | $R^2$ | $R^3$ | physikalische Eigenschaften |
|---|---|---|---|---|---|---|---|---|
| 20 | H | H | $NO_2$ | H | H | $-O-C_2H_5$ | $CF_3$ | Fp.: 90°C |
| 21 | H | $NO_2$<br>(H) | H<br>($NO_2$) | H | H | $-O-n-C_3H_7$ | $CF_3$ | $^1H$-NMR*): 7.80-8.70; A: 5.80 B: 5.85 |
| 22 | H | $NO_2$<br>(H) | H<br>($NO_2$) | H | H | 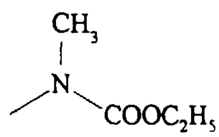 | $CF_3$ | $^1H$-NMR*): 7.95-8.80; A: 5.96 B: 5.99 |
| 23 | H | $NO_2$<br>(H) | H<br>($NO_2$) | H | H | 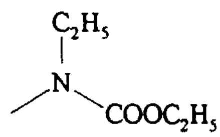 | $CF_3$ | $^1H$-NMR*): 7.90-8.75; A: 5.97 B: 5.38 |
| 24 | H | $NO_2$<br>(H) | H<br>($NO_2$) | H | H | 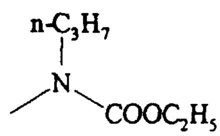 | $CF_3$ | $^1H$-NMR*): 7.91-8.80; A: 5.97 B: 5.99 |
| 25 | H | $NO_2$<br>(H) | H<br>($NO_2$) | H | H | OH | $CF_3$ | $^1H$-NMR*): 7.85-8.9; A: 5.85 B: 5.89 |
| 26 | H | F<br>(H) | H<br>(F) | H | H | $-O-C_2H_5$ | $CF_3$ | $^1H$-NMR*): A: 5.63; 7.10-7.90 B: 5.69 |
| 27 | H | 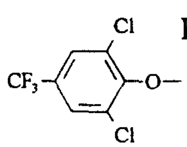<br>(H) | H<br>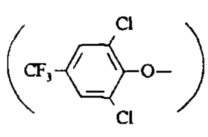 | H | H | $-O-C_2H_5$ | $CF_3$ | $^1H$-NMR*): 5.61; 5.68; 6.91-7.85 |

| Bsp.-Nr. | $X^1$ | $X^2$ | $X^3$ | $X^4$ | $R^1$ | $R^2$ | $R^3$ | physikalische Eigenschaften |
|---|---|---|---|---|---|---|---|---|
| 28 | H | 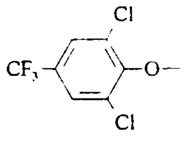 (H) | H 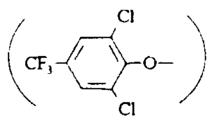 | H | H | -O-n-$C_3H_7$ | $CF_3$ | $^1$H-NMR*): 5.63; 5.79; 6.82-7.86 |
| 29 | H | 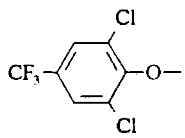 (H) | H 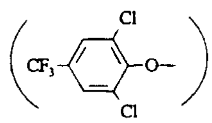 | H | H | 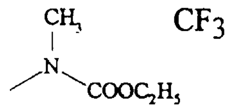 | $CF_3$ | $^1$H-NMR*): 5.84; 5.86; 6.86-7.90 |
| 30 | H | 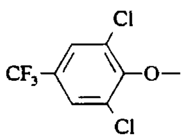 (H) | H 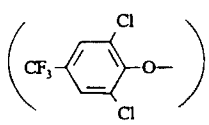 | H | H | 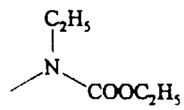 | $CF_3$ | $^1$H-NMR*): 5.80; 5.84; 6.88-7.89 |
| 31 | H | 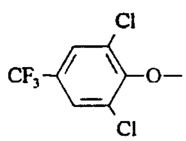 (H) | H 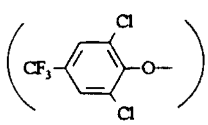 | H | H | 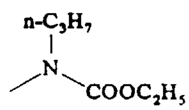 | $CF_3$ | $^1$H-NMR*): 5.78 (s), 5.88 (s), 6.88-7.95 (m) |
| 32 | H | 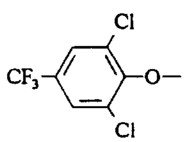 (H) | H 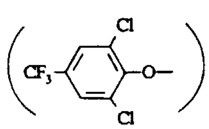 | H | H | CN | $CF_3$ | $^1$H-NMR*): 5.11; 6.95; 7.04; 7.74 ; 7.93 |
| 33 | Br | Cl | Cl | H | H | 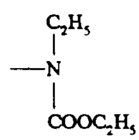 | $CF_3$ | Fp. 100-103°C |

*) Die $^1$H-NMR-Spektren wurden in Deuterochloroform ($CDCl_3$) oder Hexadeutero-Dimethylsulfoxid (DMSO-$d_6$) mit Tetramethylsilan (TMS) als innerem Standard aufgenommen. Angegeben ist die chemische Verschiebung als d-Wert in ppm.

Herstellung der Ausgangsverbindung:

Beispiel II-1:

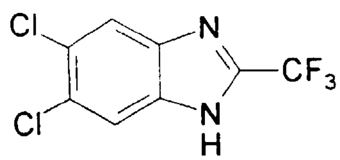

35,4 g (0,2 Mol) 4,5-Dichlorphenylen-diamin werden mit 150 ml Trifluoressigsäure für 3 Stunden auf Rückflußtemperatur erhitzt. Zur Aufarbeitung wird überschüssige Trifluoressigsäure abdestilliert und der Rückstand zwischen 100 ml Wasser und 300 ml Essigester verteilt. Die organische Phase wird abgetrennt, nacheinander mit jeweils 100 ml wässriger Natriumhydrogencarbonatlösung und Wasser gewaschen, über Natriumsulfat getrocknet und im Vakuum eingeengt. Der Rückstand wird durch Chromatographie an Kieselgel (Laufmittel: Cyclohexan/Essigester 1:1) gereinigt.

Man erhält 42,1 g (81 % der Theorie) an 5,6-Dichlor-2-trifluormethyl-1H-benzimidazol vom Schmelzpunkt 225-230°C.

In entsprechender Weise erhält man die folgenden 1H-Benzimidazole der Formel (II):

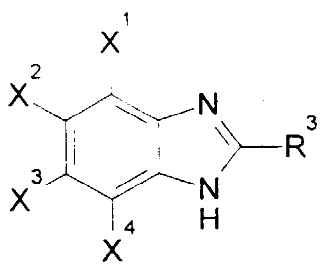

(II)

| Bsp.-Nr. | $X^1$ | $X^2$ | $X^3$ | $X^4$ | $R^3$ | physikalische Eigenschaften |
|---|---|---|---|---|---|---|
| II-2 | H | 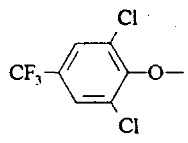 (H) | H 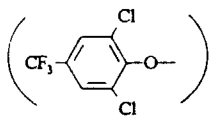 | H | $CF_3$ | Fp. 227°C |
| II-3 | H | F (H) | H (F) | H | $CF_3$ | Fp. 213°C |
| II-4 | H | $NO_2$ (H) | H ($NO_2$) | H | $CF_3$ | Fp. 151°C |
| II-5 | H | Cl (H) | H (Cl) | H | $CF_3$ | Fp. 193°C |
| II-6 | Cl (H) | H (Cl) | Cl (H) | H (Cl) | $CF_3$ | Fp. 165-170°C |
| II-7 | Br | (Cl) | Cl | H | $CF_3$ | Fp. 195-149°C |

*) Die $^1$H-NMR-Spektren wurden in Deuterochloroform ($CDCl_3$) oder Hexadeutero-Dimethylsulfoxid (DMSO-$d_6$) mit Tetramethylsilan (TMS) als innerem Standard aufgenommen. Angegeben ist die chemische Verschiebung als d-Wert in ppm.

Chlor-(2-halogen-1-fluormethyl-ethoxy)-methane der Formel

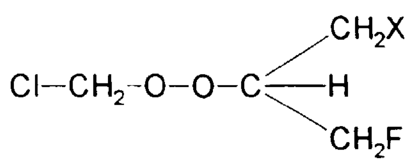

in der

X für Fluor oder Chlor steht,

[Im einzelnen handelt es sich dabei um Chlor-(2-fluor-1-fluormethyl-ethoxy)-methan (Formel (I), X = Fluor) und Chlor-(2-chlor-1-fluormethyl-ethoxy)-methan (Formel (I), X = Chlor).]
sind erhältlich durch Umsetzung von halogenierten Isopropanolen der Formel

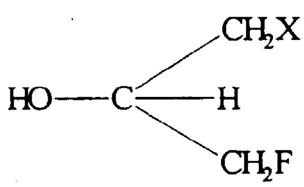

in der

X für Fluor oder Chlor steht,

bei -20 bis +20°C mit Formaldehyd und Chlorwasserstoff.

Sie dienen zur Herstellung von substituierten Benzimidazolen der Formel

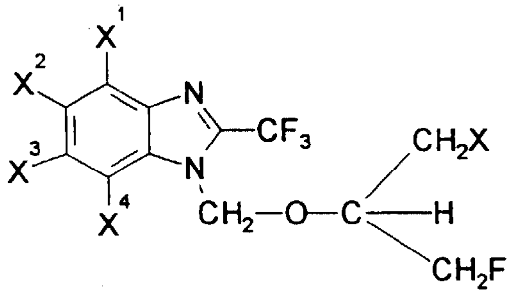

in der

X für Fluor oder Chlor stehen und

$X^1$, $X^2$, $X^3$ und $X^4$ unabhängig voneinander jeweils für Wasserstoff, Halogen, Cyano, Nitro, für jeweils gegebenenfalls substituiertes Alkyl, Alkoxy, Alkylthio, Alkylsulfinyl, Alkylsulfonyl oder Cycloalkyl, für gegebenenfalls substituiertes, ankondensienes Dioxyalkylen, für Hydroxycarbonyl, Alkylcarbonyl, Alkoxycarbonyl, Cycloalkyloxycarbonyl, für jeweils gegebenenfalls substituienes Arnino oder Aminocarbonyl oder für jeweils gegebenenfalls substituiertes Aryl, Aryloxy, Arylthio, Arylsulfinyl, Arylsulfonyl, Arylsulfonyloxy, Arylcarbonyl, Aryloxycarbonyl, Arylazo oder Arylthiomethylsulfonyl stehen, wobei jedoch mindestens einer der Substituenten $X^1$, $X^2$, $X^3$ oder $X^4$ für Halogenalkyl mit Ausnahme des Chlormethylrestes, Halogenalkoxy, Halogenalkylthio, Halogenalkylsulfinyl, Halogenalkylsulfonyl, Alkylsulfonyl, für gegebenenfalls substituiertes ankondensiertes Dioxyalkylen, für Hydroxycarbonyl, Alkylcarbonyl, Alkoxycarbonyl, Cycloalkyloxycarbonyl, für jeweils gegebenenfalls substituiertes Amino oder Aminocarbonyl oder für jeweils gegebenenfalls substituiertes Aryl, Arylthio, Arylsulfinyl, Arylsulfonyl, Arylsulfonyloxy, Arylcarbonyl, Aryloxycarbonyl, Arylazo oder Arylthiomethylsulfonyl stehen,

aus Benzimidazolen der Formel

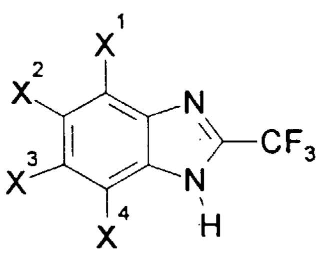

Beispiel

192 g 1,3-Difluor-2-propanol wurden mit 66 g Paraformaldehyd (fein gepulvert) versetzt. Dann wurde bei -10°C ein kräftiger Chlorwasserstoff-Gasstrom unter Rühren eingeleitet, bis eine klare 2-phasige Mischung entstanden war. Anschließend wurde die organische Phase abgetrennt, mit Calciumchlorid getrocknet und im Vakuum fraktioniert destilliert. Mit einem Siedepunkt von 50 bis 54°C bei 20 mbar wurden 183 g (60 % der Theorie) Chlor-(2-fluor-1-fluormethyl-ethoxy)-methan erhalten. Die charakteristischen Absorptionen in den NMR-Spektren waren wie folgt:

$^1$H-NMR: 5,6 ppm und 4,55 ppm.

$^{19}$F-NMR: -233 ppm.

Fluorierte 1,3-Benzo-dioxole der Formel

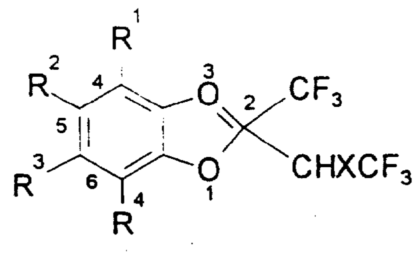

in der

X für Wasserstoff, Fluor, Chlor oder Brom stehen und

$R^1$ und $R^4$ gleich oder verschieden voneinander sein können und jeweils Wasserstoff, Halogen, $C_1$-$C_6$-Alkyl, $C_1$-$C_6$-Alkoxy,, Halogen-$C_1$-$C_6$-alkyl, $C_6$-$C_{10}$-Aryl, COOH, CN, NCO, COO-$C_1$-$C_6$-Alkyl, NH-$C_1$-$C_6$-Alkyl, N($C_1$-$C_6$-Alkyl)$_2$, bedeuten,

$R^2$ und $R^3$ für $NO_2$ oder $NH_2$ stehen,

sind erhältlich durch Umsetzung von 1,2-Dihydroxybenzolen

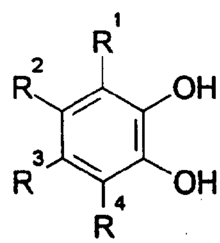

in der

$R^1$ bis $R^4$ die oben angegebene Bedeutung haben, $R^1$ bis $R^3$ jedoch nicht für OH, COCl oder $SO_2Cl$ stehen,

in Gegenwart einer Base und eines Verdünnungsmittels bei -20 bis +200°C mit einem Hexafluorbuten der Formel

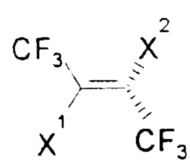

cis-trans

in der

$X^1$ für Wasserstoff oder Halogen und

$X^2$ für Halogen stehen,

oder indem man mit einer Schutzgruppe versehene 1,2-Dihydroxybenzole der Formel

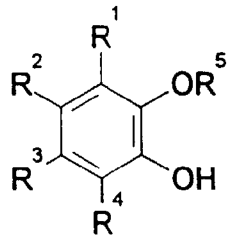

in der

$R^1$ bis $R^4$ die oben angegebene Bedeutung haben und

$R^5$ für eine Schutzgruppe oer

$R^5$ gemeinsam mit $R^1$ für einen $-C(CH_3)_2-O$-Rest stehen,

zunächst mit einem Hexafluorbuten der Formel umsetzt

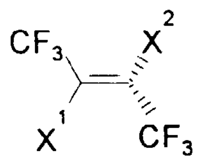

cis-trans

in der

$X^1$ für Wasserstoff oder Halogen und

$X^2$ fur Halogen stehen,

so ein Zwischenprodukt der Formel erhält,

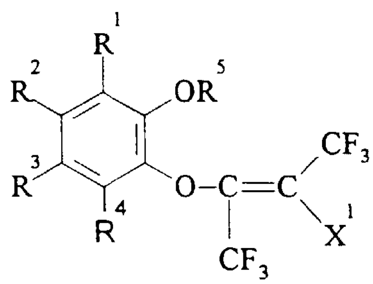

in der

$X^1$, $R^1$ bis $R^5$ die oben angegebene Bedeutung haben

aus dem Zwischenprodukt der obigen Formel die Schutzgruppe $R^5$ abspaltet,
die so erhältliche OH-Verbindung mit einer Base umsetzt und so 1,3-Benzo-dioxole der obigen Formel erhält.

1,3-Benzo-dioxole, die zwei benachbane Aminogruppen enthalten, können mit Trifluoressigsäure in das entsprechende Benzimidazol z.B. der folgenden Formel überführt werden

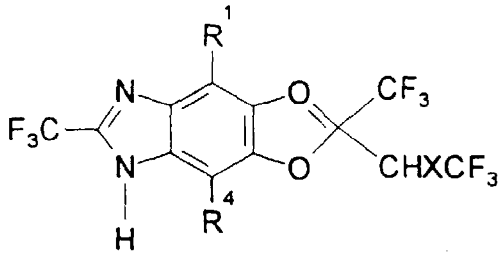

in der
$R^1$, $R^4$ und X die oben angegebene Bedeutung haben.

Aus diesen kann man durch Alkylierung Benzimidazolderivate erhalten, die im Stickstoffatom mit einem

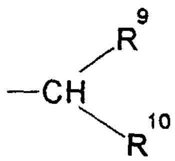

-Rest

substituiert sind.

Beispiele

Beispiel 1 a

2-(2,2,2-Trifluorethyl)-2-trifluormethyl-1,3benzodioxol

11 g Brenzkatechin wurden in 200 ml Dimethylfomamid gelöst und mit 18 g 45 gew.-%iger wäßriger Natronlauge versetzt. Die Mischung wurde bei 75°C tropfenweise mit 20g 2-Chlor-1,1,1,4,4,4-hexafluor-2-buten versetzt. Man rührte 30 Minuten bei 75°C nach. Anschließend wurde der Ansatz auf 500 ml Eiswasser gegossen und mit Diethylether extrahiert. Die organische Phase wurde mit Wasser gewaschen, mit Magnesiumsulfat getrocknet und eingeengt. Das Produkt wurde schließlich im Hochvakuum destilliert. Die Ausbeute betrug 15 g (= 56 %), der Siedepunkt 60°C bei 10 mbar. Die NMR-Spektren zeigten folgende charakteristische Absorptionen: $^{19}F$-NMR: -59,0 und -84,6 ppm. $^1H$-NMR: 3,02 ppm.

Beispiel 2 a

2-(1-Chlor-2,2,2-trifluorethyl)-2-trifluormethyl-1,3-benzodioxol

110 g Brenzkatechin wurden in 1 500 ml Acetonitril gelöst und mit 200 g Triethylamin versetzt. Die Mischung wurde bei 75°C tropfenweise mit 235 g 2,3-Dichlor-1,1,1,4,4,4-hexafluor-2-buten versetzt. Man rührte 2 Stunden bei 75°C nach. Anschließend wurden 1 200 ml des Lösungsmittels im Vakuum abdestilliert und der Rückstand mit 1 500 ml Wasser aufgenommen. Das Produkt wurde mit Diethylether extrahiert, die organische Phase 2 mal mit 10 gew.-%iger wäßriger Natronlauge und 1 mal mit Wasser gewaschen. Nach dem Trocknen mit Magnesiumsulfat wurde eingeengt und im Vakuum fraktioniert destilliert. Die Ausbeute betrug 258 g (= 84 % der Theorie). Der Siedepunkt lag bei 63°C bei 12 mbar. Die NMR-Spektren zeigten folgende charakteristische Absorptionen: $^{19}F$-NMR: -66,8 und -79,7 ppm. $^1H$-NMR: 4,71 ppm.

Beispiele 3a

2-(1,1,1,4,4,4-Hexafluor-2-butenoxy)-methoxybenzol

260 g 2-Methoxyphenol wurden in 1 l Dimethylformamid(technische Qualität) gelöst und mit 220 g 45-%iger Natronlauge versetzt. Dann wurden bei 22°C unter Rühren 400 g 2-Chlor-1,1,1,4,4,4-hexafluor-2-buten zugetropft. Man rührte 2 Stunden bei 22°C nach. Dann versetzte man mit 1,5 l Eiswasser und extrahierte mit Methylenchlorid.

Die vereinigten organischen Phasen wurden 2 mal mit 10-%iger Natronlauge und 1 mal mit gesättigter NaCl-

Lösung gewaschen, mit $MgSO_4$ getrocknet und destilliert. Die Ausbeute betrug 329 g (58 % der Theorie), der Siedepunkt 68-70°C bei 12 mbar. Die NMR-Spektren zeigten folgende charakteristische Absorptionen: $^{19}F$-NMR: -57,6 und -67,9 ppm. $^{1}H$-NMR: 5,92 ppm.

Beispiel 4a

2-(1,1,1,4,4,4-Hexafluor-2-butenoxy)-phenol

286,1 g 2-(1,1,1,4,4,4-Hexafluor-2-butenoxy)-methoxybenzol aus Beispiel 3a wurden in einer Mischung aus 500 ml Eisessig und 500 ml 48-%iger Bromwasserstoffsäure gelöst und mit 5 g Triethylbenzylammoniumchlorid versetzt. Die Mischung wurde bei 150°C Badtemperatur gerührt bis gemäß gaschromatografischer Kontrolle ein vollständiger Umsatz erreicht war. Dann ließ man abkühlen und versetzte mit 2 kg Eiswasser. Die wäßrige Phase wurde mit $CH_2Cl_2$ gründlich extrahiert. Nach Trocknen mit $MgSO_4$ wurde das Lösungsmittel abgezogen und der Rückstand im Vakuum destilliert. Die Ausbeute betrug 200 g (50 % der Theorie), der Siedepunkt 80°C bei 16 mbar. Die NMR-Spektren zeigten folgende charakteristische Absorptionen: $^{19}F$-NMR: -59,6 und -69,6 ppm. $^{1}H$-NMR: 6,1 ppm.

Beispiel 5a

2-(2,2,2-Trifluorethyl)-2-trifluormethyl-1,3-benzodioxol

200 g 2-(1,1,1,4,4,4-Hexafluor-2-butenoxy)-phenol aus Beispiel 4a wurden in 400 ml Acetonitril gelöst und mit 5 g Triethylamin versetzt. Die Mischung wurde 4 h bei 70°C gerührt. Dann wurde im Vakuum destilliert. Die Ausbeute betrug 162 g (81 % der Theorie), der Siedepunkt 60°C bei 10 mbar. Die NMR-Spektren zeigten folgende charakteristische Absorptionen: $^{19}F$-NMR: -59,0 und -84,6 ppm. $^{1}$-H-NMR: 3,02 ppm.

Beispiel 6a

2-(2-Chlor-1,1,1,4,4,4-hexafluor-2-butenoxy)-1-benzyloxybenzol

20 g 2-Benzyloxyphenol wurden in 100 ml Dimethylformamid gelöst und mit 9 g 45 %iger Natronlauge versetzt. Dann wurde bei Raumtemperatur 23 g 2,3-Dichlor-1,1,1,4,4,4-hexafluor-2-buten zugetropft. Nach Abklingen der exothermen Reaktion wurde 1 Stunde bei Raumtemperatur nachgerührt. auf Wasser gegeben und mit tert.-Butylmethylether extrahiert. Nach Trocknen mit $MgSO_4$ wurde das Lösungsmittel abgezogen. Die Ausbeute betrug 29g (74% der Theorie). Die NMR-Spektren zeigten folgende charakteristische Absorptionen: $^{19}F$-NMR: -59,5; -60,5; -61,7 und -62,8 ppm.

Beispiel 7a

2-(2-Chlor-1,1,1,4,4,4-hexafluor-2-butenoxy)-phenol

24,4 g 2-(2-Chlor-1,1,1,4,4,4-hexafluor-2-butenoxy)-1-benzyloxybenzol aus Beispiel 6a wurden in 150 ml Tetrahydrofuran gelöst und bei Raumtemperatur 4 Stunden mit 3 bar Wasserstoff in Gegenwart von 2 g Pd/C (10 %ig) behandelt. Anschließend wurde filtriert, eingeengt und im Vakuum destilliert. Die Ausbeute betrug 13,2 g (69 % der Theorie), der Siedepunkt 56°C bei 0,15 mbar.

Beispiel 8a

2-(1-Chlor-2,2,2-trifluorethyl)-2-trifluormethyl-1,3-benzodioxol

11,7 g 2-(2-Chlor-1,1,1,4,4,4-hexafluor-2-butenoxy)-phenol aus Beispiel 7a wurden in 40 ml tert.-Butylmethylether gelöst und mit 40 ml ln-Natronlauge versetzt. Nach 30-minütigem Rühren bei Raumtemperatur wurde die organische Phase abgetrennt, mit $MgSO_4$ getrocknet und destilliert. Die Ausbeute betrug 10 g (88 % der Theorie), der Siedepunkt 63°C bei 12 mbar. Die NMR-Spektren zeigten folgende charakteristischeAbsorptionen: $^{19}F$-NMR: -66,8 und -79,7 ppm. $^{1}H$-NMR: 4,71 ppm.

Beispiel 9a

2,2-Dimethyl-4-(1,1,1,4,4,4-hexafluor-2-butenoxy)-1,3-benzodioxol (Formel V, $R^5$ gemeinsam mit $R^1$ = $-C(CH_3)_2-O-$ Rest)

46 g 2,2-Dimethyl-4-hydroxy-1,3-benzodioxol (Formel IV, $R^5$ gemeinsam mit $R^3$ = $-C(CH_3)_2$-O-Rest) wurden in 200ml N-Methylpyrrolidon gelöst und mit 31 g 40 gew.-%iger wäßriger Natronlauge versetzt. Dann wurde unter Rühren bei Raumtemperatur 54,8 g 2-Chlor-1,1,1,4,4,4-hexafluor-2-buten tropfenweise hinzugefügt. Nach 1 Stunde Nachrühren wurde der Ansatz auf Wasser gegossen und mit tert.-Butylmethylether extrahiert. Die organische Phase wurde mit 10 gew.-%iger wäßriger Natronlauge gewaschen, mit Magnesiumsulfat getrocknet und am Rotationsverdampfer die leicht flüchtigen Anteile entfernt. Es hinterblieben 73,8 g (= 80 % der Theorie) eines gemäß Gaschromatographie 95 % reinen Produktes. Die charakteristischen Absorptionen in den NMR-Spektren waren: $^{19}$F-NMR: -58,1 und -68,5 ppm. $^{1}$H-NMR: 6,73, 6,55, 6,03 und 1,70 ppm.

Beispiel 10a

1,2-Dihydroxy-3-(1,1,1,4,4,4-hexafluor-2-butenoxy)-benzol

65 g des Produktes aus Beispiel 9a wurden mit 200 ml konzentrierter wäßriger Salzsäure 4 Stunden lang unter Rühren zum Sieden am Rückfluß erhitzt. Anschließend wurde der Ansatz mit 300 ml Wasser verdünnt und mit Methylenchlorid extrahiert. Nach dem Trocknen mit Magnesiumsulfat wurde aus der organischen Phase das Lösungsmittel abgezogen und 54 g eines zu 90 % reinen Produktes erhalten. Die Umkristallisation aus Cyclohexan ergab farblose Kristalle mit einem Schmelzpunkt von 105°C. Die charakteristischen Absorptionen in den NMR-Spektren waren wie folgt: $^{19}$F-NMR -57,7 und -67,7 ppm. $^{1}$H-NMR: 6,77, 6,50, 6,21 und 5,42 ppm.

Beispiel 11a

2-(2,2,2-Trifluorethyl)-2-(trifluormethyl)4-hydroxy-1,3-benzodioxol (Formel (I), $R^1$ = OH, X = H, A = CH, $R^2$ und $R^3$ = H).

43,5 g des Produktes aus Beispiel 10a wurden in 300 ml Acetonitril gelöst und bei Raumtemperatur mit 1,5 g Triethylamin versetzt. Nach 2 Stunden Rühren bei Raumtemperatur wurde das Lösungsmittel abgezogen und der Rückstand im Vakuum destilliert. Die Ausbeute betrug 17 g (= 39 % der Theorie), der Siedepunkt 85°C bei 0,15 mbar, der Schmelzpunkt 65°C. Die charakteristischen Absorptionen in den NMR-Spektren waren wie folgt: $^{19}$F-NMR: -59,0 und -84,5 ppm. $^{1}$H-NMR: 6,80, 6,55, 6,2 und 3,01 ppm.

Beispiel 12a

2,2-Dimethyl-4-(3-chlor-1,1,1,4,4,4-hexafluor-2-butenoxy)-1,3-benzodioxol (Formel (V), $R^1$ und $R^5$ gemeinsam $-C(CH_3)_2-O-$, $X^1$ = Cl, $R^2$ + $R^3$ = H, A = CH).

33,2 g 2,2-Dimethyl-4-hydroxy-1,3-benzodioxol wurden analog Beispiel 9a mit 47 g 2,3-Dichlor-1,1,1,4,4,4-hexafluor-2-buten umgesetzt. Das erhaltene Produkt wurde im Vakuum destilliert undein 1:1 molares Gemisch aus cis/trans-Isomeren erhalten. Die Ausbeute betrug 51 g (= 70 % der Theorie), der Siedepunkt 70°C bei 0,15 mbar. Die charakteristischen Absorptionen in den NMR-Spektren waren wie folgt: $^{19}$F-NMR: -60,0, -61,6, -62,2 und 63,4 ppm. $^{1}$H-NMR: 6,79, 6,65 bis 6,48 und 1,7 ppm.

Beispiel 13a

1,2-Dihydroxy-3-(3-chlor-1,1,1,4,4,4-hexafluor-2-butenoxy)-benzol (Formel (V), $R^1$ = OH, $R^2$ + $R^3$ = H, A = CH, $R^5$ = H, $X^1$ = Cl)

18 g des Produktes aus Beispiel 12a wurden analog Beispiel 10a mit 50 ml konzentrierter Salzsäure umgesetzt. Es wurden 15,7 g eines zu 97 % reinen Produktes erhalten. Das Produkt war ein 1:1 molares Gemisch der cis/trans-Isomere. Die charakteristischen Absorptionen in den NMR-Spektren waren wie folgt:$^{19}$F-NMR: -60,2, -61,3, -62,2 und -63,3 ppm. $^{1}$H-NMR: 6,80, 6,45 und 6,25 ppm.

Beispiel 14a

2-(1-Chlor-2,2,2-trifluorethyl)-2-trifluormethyl-4-hydroxy-1,3-benzodioxol

15 g des Produktes aus Beispiel 13a wurden in 50 ml Acetonitril gelöst und mit 1 ml Triethylamin versetzt. Nach 15 minütigem Rühren wurde das Lösungsmittel abgezogen und der Rückstand im Vakuum destilliert. Zur Reinigung wurde das Produkt mit Diethylether aufgenommen und über Siliciumdioxid filtriert. Nach dem Abziehen des Diethylethers verblieben 10,5 g des Produktes (= 70 % der Theorie). Der Schmelzpunkt betrug 139 bis 141°C. Die charakteristischen Absorptionen in den NMR-Spektren waren wie folgt: $^{19}$ F-NMR: -66,6 und -79,3 ppm. $^{1}$H-NMR: 8,4, 6,76, 6,60, 6,50 und 4,70 ppm.

Beispiel 15a

5-Nitro-2-(2,2,2-trifluorethyl)-2-trifluormethyl-1,3-benzodioxol

Eine Lösung von 54,4 g 2-(2,2,2-Trifluorethyl)-2-trifluormethyl-1,3-benzodioxol in 75 ml Methylenchlorid wurde bei 10°C zu einer Mischung aus 40 ml 65 gew.-%iger Salpetersäure und 40 ml konzentrierter Schwefelsäure getropft. Der Ansatz wurde 1 Stunde bei Raumtemperatur nachgerührt, dann auf Eiswasser gegossen, die organische Phase abgetrennt und die wäßrige Phase mit Methylenchlorid extrahiert. Die vereinigten organischen Phasen wurden mit Wasser gewaschen, getrocknet und von leichtflüchtigen Bestandteilen befreit. Es hinterblieben 95 g des Produktes (= 86 % der Theorie) mit einem Schmelzpunkt von 87 bis 88°C.

Die NMR-Spektren zeigten folgende charakteristischen Absorptionen: $^{19}$F-NMR: -59,0 und -69,4 ppm. $^{1}$H-NMR: 3.10 ppm.

Beispiel 16a

5-Nitro-2-(1-chlor-2,2,2-trifluorethyl)-2-trifluormethyl-1,3-benzodioxol

613 g 2-(1-Chlor-2,2,2-trifluorethyl)-2-trifluormethyl-1,3-benzodioxol aus Beispiel 2a wurden in 1,2 l Methylenchlorid gelöst und bei 0 bis 10°C zu einer Mischung aus 400 ml 65 %iger Salpetersäure und 400 ml konz. Schwefelsäure getropft. Man rührte 2 Stunden bei Raumtemperatur nach. Dann wurde vorsichtig auf 2 l Eiswasser gegeben und mit Methylenchlorid extrahiert. Die vereinigten organischen Phasen wurden 2 mal mit Wasser gewaschen, getrocknet und eingeengt. Die Ausbeute betrug 652 g (93 % der Theorie). Die NMR-Spektren zeigten folgende charakteristische Absorptionen: $^{19}$F-NMR: -66,4 und -79,2 ppm. $^{1}$H-NMR: 4,81 ppm.

Beispiel 17a

5,6-Dinitro-2-(2,2,2-trifluorethyl)-2-trifluormethyl-1,3-benzodioxol

317 g des Produktes aus Beispiel 15a wurden vorgelegt und dazu unter Rühren eine Mischung aus 250 ml 100 gew.-%iger Salpetersäure und 350 ml konzentrierter Schwefelsäure zugetropft. Die Mischung wurde 2 Stunden bei 55°C gerührt. Dann ließ man den Ansatz abkühlen und goß ihn auf Eiswasser. Das Produkt wurde mit Methylenchlorid extrahiert, mit Natriumhydrogencarbonatlösung neutral gewaschen, getrocknet und am Rotationsverdampfer von leicht flüchtigen Bestandteilen befreit. Die Ausbeute betrug 339 g (= 94 % der Theorie), der Schmelzpunkt 101 bis 103°C.

Die NMR-Spektren zeigten folgende charakteristischen Absorptionen: $^{19}$F-NMR: -60,9 und -86,5 ppm. $^{1}$H-NMR: 3,18 ppm.

Beispiel 18a

5,6-Dinitro-2-(1-Chlor-2,2,2-trifluorethyl)-2-trifluormethyl-1,3-benzodioxol

352 g 5-Nitro-2-(1-chlor-2,2,2-trifluorethyl)-2-trifluormethyl-1,3-benzodioxol aus Beispiel 16a wurden vorgelegt und mit einer Mischung aus 250 ml 100 gew.-%iger Salpetersäure und 350 ml konzentrierter Schwefelsäure versetzt. Die Mischung wurde 2 Stunden bei 60°C gerührt. Nach dem Abkühlen goß man auf Eiswasser und extrahierte mit Methylenchlorid. Nach Waschen mit Natriumhydrogencarbonatlösung und Trocknen wurde einrotiert. Die Ausbeute betrug 392 g (91 % der Theorie), der Schmelzpunkt 125°C. Die NMR-Spektren zeigten folgende charakteristische Absorptionen: $^{19}$F-NMR: -68,5 und -81,0 ppm. $^{1}$H-NMR: 4,86 ppm.

Beispiel 19a

5-Amino-2-(2,2,2-trifluorethyl)-2-trifluormethyl-1,3-benzodioxol

57,4 g des Produktes aus Beispiel 15a wurden in 400 ml Tetrahydrofuran gelöst und in Gegenwart von 4 g Katalysator (Palladium auf Kohle, 10gew.-%ig) 5 Stunden bei 30°C bei 50 bar mit Wasserstoff hydriert. Danach wurde abfiltriert, das Lösungsmittel entfernt und im Hochvakuum destilliert. Es wurden 37 g Produkt (= 63 % der Theorie) mit einem Siedepunkt von 83°C bei 0,07 mbar erhalten. $^{19}F$-NMR: -59,0 und -84,6 ppm. $^{1}H$-NMR: 2,98 ppm.

Beispiel 20a

5-Amino-2-(1-chlor-2,2,2-trifluorethyl)-2-trifluormethyl-1,3-benzodioxol

72 g 5-Nitro-2-(1-chlor-2,2,2-trifluorethyl)-2-trifluormethyl-1,3-benzodioxol aus Beispiel 16a wurden in 500 ml Tetrahydrofuran gelöst und an 5 g Palladium auf Kohle (5 %ig) 5 Stunden bei Raumtemperatur mit 15 bis 20 bar Wasserstoff hydriert. Anschließend wurde filtriert und das Lösungsmittel im Vakuum abgezogen. Die Ausbeute betrug 60 g (93 % der Theorie), der Siedepunkt 80 bis 82°C bei 0,1 mbar. Die NMR-Spektren zeigten folgende charakteristische Absorptionen: $^{19}F$-NMR: -66,5 und -79,4 ppm. $^{1}H$-NMR: 4,68 ppm.

Beispiel 21a

5,6-Diamino-2-(2,2,2-trifluorethyl)-2-trifluormethyl-1,3-benzodioxol

339 g des Produktes aus Beispiel 17a wurden in 2 000 ml Tetrahydrofuran gelöst und mit 20 g Katalysator (Palladium auf Kohle, 5 gew.-%ig) versetzt. Bei 25 bis 30 bar wurde 13 Stunden lang bei Raumtemperatur mit Wasserstoff hydriert. Anschließend wurde der Ansatz abfiltriert und das Lösungsmittel im Vakuum abgezogen. Es blieb ein Feststoff zurück. Die Ausbeute betrug 274 g (= 96 % der Theorie). $^{19}$ F-NMR: -61,2 und -86,6 ppm. $^{1}H$-NMR: 3,02 ppm.

Beispiel 22a

2-(2,2,2-Trifluorethyl)-2-trifluormethyl-1,3-benzodioxol

306,5 g 2-(1-Chlor-2,2,2-trifluorethyl)-2-trifluormethyl-1,3-benzodioxol aus Beispiel 2a wurden in 500 ml THF gelöst und mit 101 g Triethylamin und 30 g Palladium auf Kohle (5 gew.-%ig) versetzt. Dann wurde 48 h bei 110°C mit 100 bar Wasserstoff hydriert. Anschließend wurde filtriert, das Lösungsmittel abgezogen und der Rückstand im Vakuum fraktioniert. Die Ausbeute betrug 126 g (46 % der Theorie), der Siedepunkt 60°C bei 10 mbar. Die NMR-Spektren zeigten folgende charakteristische Absorption: $^{19}F$-NMR: -59,0 und -84,6 ppm. $^{1}H$-NMR: 3,02 ppm.

Fluoralkyl(en)gruppen enthaltende o-Phenylendiamine der Formel

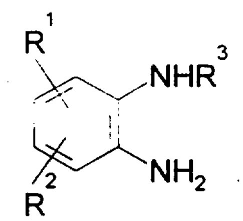

in der

$R^1$ für $CF_3$, $OCF_3$, $SCF_3$, $SO_2$-$C_1$-$C_6$-Alkyl, das geradkettig oder verzweigt und durch Fluor ganz oder teilweise substituiert sein kann, $N(CF_3)_2$, einen Phenyl- oder Phenoxyrest mit $CF_3$ oder CN in 4-Position und gegebenenfalls weiteren Substituenten, 1,1,2,3,3,3-Hexafluorpropoxy, 1,1,2-Trifluor-2-chlor-ethoxy, 1,1,2,2-Tetrafluorethoxy, 1,1,2-Trifluor-2-chlor-ethylthio oder 1,1,2,3,3,3-Hexafluorpropylthio, unabhängig davon

$R^2$ für F, Cl, Br, CN, $CH_3$, $OCF_3$, $SO_2$-$C_1$-$C_6$-Alkyl, das geradkettig oder verzweigt und durch Fluor ganz

oder teilweise substituiert sein kann, $COO-C_1-C_6$-Alkyl, $COOC_6H_5$, 1,1,2,2-Tetrafluorethoxy, 1,1,2,3,3,3-Hexafluorpropoxy oder 1,1,2-Trifluor-2-chlor-ethoxy und

$R^3$ für Wasserstoff, $COCH_3$ oder $COCF_3$ stehen, wobei

$R^1$ und $R^2$ gemeinsam für einen -O-CFCl-CFCl-O-Rest stehen können,

mit Ausnahme der in der EP-A 251 013 und der EP-A 487 286 beschriebenen Verbindungen sind erhältlich, indem man ein Benzolderivat der Formel

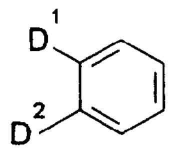

in der

$D^1$ für $CF_3O$, $CF_3S$, $CHF_2CF_2O$, $CHFCl-CF_2O$, $CF_3CHFCF_2O$, $CF_3CF_2O$, $CF_3CF_2CF_2O$, $CF_3CF_2S$ oder $CF_3CHFCF_2O$ und

$D^2$ für $CF_3O$, $CF_3S$, $CHF_2CF_2O$, $CHFCl-CF_2O$, $CF_3CHF-CF_2O$, $CF_3CF_2O$, $CF_3CF_2CF_2O$, $CF_3CF_2S$, $CF_3CHFCF_2O$, Fluor, Chlor, Brom, $C_1-C_6$-Alkyl oder $C_1-C_6$-Alkoxy

steht
dinitriert, die Nitrogruppen anschließend reduziert und so Verbindungen erhält, bei denen $R^1$ und $R^2$ in 4- und 5-Stellung zu den Aminogruppen stehen und die Bedeutung von $D^1$ und $D^2$ haben.

Sollen Verbindungen hergestellt werden, bei denen $R^1$ die oben angegebene Bedeutung hat und in 4-Stellung zu den Aminogruppen steht und $R^2$ für Cl oder Br in 5-Stellung zu den Aminogruppen steht, so kann man z.B. ein Nitrobenzolderivat der Formel

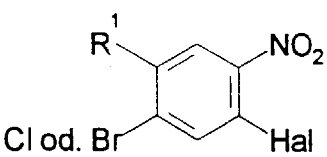

in der

$R^1$ die angegebene Bedeutung hat und

Hal für Fluor, Chlor oder Brom steht,

mit Ammoniak umsetzen, so die Hal-Gruppe gegen eine Aminogruppe austauschen und das so erhaltene Nitranilin reduzieren.

Sollen Verbindungen hergestellt werden, bei denen $R^1$ die oben angegebene Bedeutung hat und in 4-Stellung zu den Aminogruppen steht, $R^2$ für Chlor oder Brom in 6-Stellung zu den Aminogruppen steht und $R^3$ Wasserstoff bedeutet, so kann man z.B. ein Nitranilin der Formel

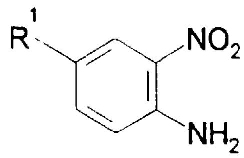

in der

$R^1$ die oben angegebene Bedeutung hat.

mit einem Chlorierungs- oder Bromierungsmittel umsetzen, so ein Chlor- oder Bromatom in die meta-Stellung zur Nitrogruppe einführen und anschließend die Nitrogruppe reduzieren.

Sollen Verbindungen hergestellt werden, bei denen $R^1$ eine Donorgruppe in 4-Stellung zu den beiden Aminogruppen, $R^2$ eine Akzeptorgruppe, z.B. COO-$C_1$-$C_6$-Alkyl, CN, $CF_3$ oder $SO_2$-$C_1$-$C_6$-Alkyl darstellt und $R_3$ ungleich Wasserstoff ist, so kann man z.B. ein Benzolderivat der Formel

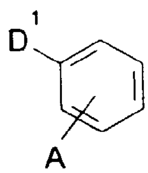

in der

$D^1$ die oben angegebene Bedeutung hat und

A für $CF_3$, $SO_2$-$C_1$-$C_6$-Alkyl, das geradkettig oder verzweigt und durch Fluor ganz oder teilweise substituiert sein kann, COO-$C_1$-$C_6$-Alkyl oder CN steht,

mononitrieren (Eintritt der $NO_2$-Gruppe in para-Position zu $D^1$), die$NO_2$-Gruppe zur $NH_2$-Gruppe reduzieren, die $NH_2$-Gruppe z.B. mit Essigsäure oder Trifluoressigsäure acylieren, nochmals monomitrieren (Eintritt dieser $NO_2$-Gruppe in ortho-Position zur NHCOR-Gruppen mit R = z.B. $CH_3$ oder $CF_3$), diese $NO_2$-Gruppe zur $NH_2$-Gruppe reduzieren und gegebenenfalls, wenn man eine Verbindung der obigen Formel mit $R^3$ = Wasserstoff herstellen will, die Acylgruppe durch Verseifung abspalten.

Die Fluoralkyl(en)gruppen enthaltenden o-Phenylendiamine, in denen $R^3$ Wasserstoff bedeutet, können zunächst mit Trifluoressigsäure zu 2-Trifluormethylbenzimidazolen der Formel

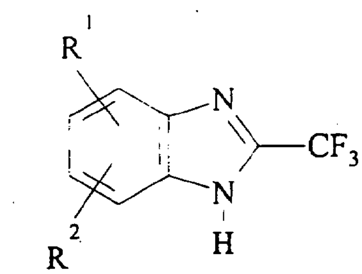

umgesetzt und dann weiter mit Verbindungen der Formel

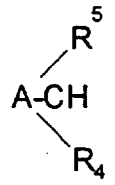

umgesetzt werden, wobei $R^1$ und $R^2$ den obigen Bedeutungsumfang annehmen,

$R^4$ für Wasserstoff, Alkyl, Alkoxy oder für gegebenenfalls substituiertes Aryl steht,

$R^5$ für Hydroxy, Cyano oder für jeweils gegebenenfalls substituiertes Alkyl, Alkenyl, Alkinyl, Alkoxy, Alkenyloxy, Alkinyloxy, Alkylthio, Amino, Aminocarbonyl, Alkylcarbonyl, Alkoxycarbonyl, Alkylcarbonyloxy, Dialkoxyphosphonyl, (Hetero)Aryl, (Hetero)Arylcarbonyl, (Hetero)Aryloxycarbonyl, (Hetero)Arylcarbonyloxy oder (Hetero)Arylaminocarbonylaminocarbonyloxy steht und

A eine geeignete Abgangsgruppe bedeutet.

Abgangsgruppen sind dem Fachmann bekannt und sind beispielsweise Halogen, Alkyl(Alkoxy, Aryl)sulfonyloxy, Hydroxy oder Alkoxy.

Beispiele

Beispiele 1 bis 6 b (Dinitrierung und Reduktion)

Beispiel 1 b

Zu 500 g einer Mischsäure enthaltend 33 Gew.-% $HNO_3$ und 67 Gew.-% $H_2SO_4$ wurden 320g 1,2-Bis-(2-chlor-1,1,2-trifluorethoxy)-benzol getropft. Nach einer Stunde bei 40°C wurden 250 ml 20 gew.-%iges Oleum zugetropft. Anschließend wurde auf 80°C erhitzt und 15 Stunden lang nachgerührt. Dann wurden weitere 120 ml 20 gew.-%iges Oleum und 250 g der oben angegebenen Mischsäure zugetropft. Nach 6 Stunden bei 80 bis 82°C wurde abgekühlt und auf Eis gegossen. Die organische Phase wurde abgetrennt und mit Wasser gewaschen. Nach azeotroper Trocknung mit 1,2-Dichlorethan wurden 350 g 96 Gew.-% reines 1,2-Dinitro-4,5-bis-(2-chlor-1,1,2-trifluorethoxy)-benzol erhalten (Öl, $n_D^{20}$: 1,4832, GC 99,1 %)

350 g dieser Dinitroverbindung wurden zu einem Gemisch aus 1,5 l Ethanol, 50 ml Wasser, 30 ml konzentrierter wäßriger Salzsäure und 470 g Eisenspänen getropft und insgesamt 15 Stunden zum Sieden am Rückfluß erhitzt. Danach wurde die erkaltete Lösung abfiltriert, eingeengt und der Rückstand aus Cyclohexan umkristallisiert. Es wurden 216 g 1,2-Diamino-4,5-bis-(2-chlor-1,1,2-trifluorethoxy)-benzol mit einem Schmelzpunkt von 58 bis 60°C erhalten.

Beispiel 2b

Analog Beispiel 1 wurde aus 1,2-Bis-(1,1,2,3,3,3-hexafluorpropoxy)-benzol die entsprechende 4,5-Dinitroverbindung (Öl, $n_D^{20}$: 1,4852) und die entsprechende 4,5-Diaminoverbindung (Öl, 87 Gew.-% rein) hergestellt.

Beispiel 3 b

Analog Beispiel 1 wurde aus 1-(1,1,2-Trifluor-2-chlorethoxy)-2-chlorbenzol die entsprechende 4,5-Dinitroverbindung (Schmelzpunkt 56 bis 57°C) und die entsprechende 4,5-Diaminoverbindung (Schmelzpunkt 67 bis 68°C) hergestellt.

Beispiel 4 b

Analog Beispiel 1 wurde aus 1-Trifluormethoxy-2-brombenzol die entsprechende 4,5-Dinitroverbindung (Schmelzpunkt 73 bis 75°C) und die entsprechende 4,5-Diaminoverbindung (Öl, 98 Gew.-% rein, $n_D^{20}$: 1,5485) hergestellt.

Beispiel 5 b

Analog Beispiel 1 wurde aus l-Trifluormethoxy-2-chlorbenzol die entsprechende 4,5-Dinitroverbindung (Schmelzpunkt 55 bis 56°C) und die entsprechende 4,5-Diaminoverbindung (Schmelzpunkt 56 - 57°C) hergestellt.

Beispiel 6 b

Aus 1-(1,1,2,3,3,3-Hexafluorpropoxy)-2-chlor-benzol wurde die entsprechende 4,5-Dinitroverbindung (Öl) und die entsprechende 4,5-Diaminoverbindung (Öl) hergestellt.

Beispiele 7 bis 12 b

Verdrückung mit Ammoniak und Reduktion

Beispiel 7 b

In einem Autoklaven wurden 260 g 3-Nitro-2,5-dichlorbenzotrifluorid, 130 ml Wasser und 10 g Tetraethylammoniumchlorid vorgelegt und 120 ml flüssiges Ammoniak aufgedrückt. Anschließend wurde auf 130°C erhitzt und für 10 Stunden bei dieser Temperatur gerührt. Nach dem Abkühlen wurde der Ansatz abfiltriert, der abgetrennte Niederschlag mit Wasser gewaschen und getrocknet. Es fielen 194 g 2-Amino-3-nitro-5-chlor-benzotrifluorid mit einem Schmelzpunkt von 67°C an.

134 g des wie oben beschrieben erhaltenen Nitranilins wurden in 800 ml Ethanol gelöst, dann 20 ml Wasser, 10 ml konzentrierte wäßrige Salzsäure und 160 g Eisenspäne zugegeben. Die Mischung wurde für 15 Stunden zum Sieden am Rückfluß erhitzt, dann abgekühlt, abgesaugt, der Filterrückstand mit Dichlormethan gewaschen und anschließend die organischen Phasen unter reduziertem Druck vom Lösungsmittel befreit. Es fielen 171 g 5-Chlor-3-trifluormethyl-1,2-diaminobenzol mit einem Schmelzpunkt von 53°C an.

Beispiel 8 b

Analog Beispiel 7 wurde aus 3-Nitro-4,6-dichlor-difluorchlormethoxybenzol zunächst 3-Nitro-4-amino-6-chlor-difluorchlormethoxybenzol (Schmelzpunkt 73°C) und daraus 3,4-Diamino-6-chlor-difluorchlormethoxybenzol (Öl) erhalten.

Beispiel 9 b

Analog Beispiel 7 wurde aus 3-Brom-5-nitro- 6-chlorbenzotrifluorid zunächst 3-Brom-5-nitro-6-amino-benzotrifluorid (Schmelzpunkt 80 bis 82°C) und daraus 3-Brom-5,6-diamino-benzotrifluorid (Schmelzpunkt 52bis 54°C) hergestellt.

Beispiel 10 b

Analog Beispiel 7 wurde aus 3-Cyano4-chlor-5-nitro-benzotrifluorid zunächst 3-Cyan-4-amino-5-nitro-benzotrifluorid (Schmelzpunkt 99 bis 100°C) und daraus 3-Cyano-4,5-diamino-benzotrifluorid hergestellt.

Beispiel 11 b

Analog Beispiel 7 wurde aus 3,6-Dichlor-5-nitro-benzotrifluorid zunächst 3-Chlor-5-nitro-6-amino-benzotrifluorid (Schmelzpunkt 53 bis 54°C) und daraus 3-Chlor-5,6-diamino-benzotrifluorid hergestellt.

Beispiel 12 b

Aus 2-Brom-4-fluor-5-nitro-(1,1,2-trifluor-2-chlor)-ethoxybenzol wurde zunächst 2-Brom-4-amino-5-nitro-(1,1,2-trifluor-2-chlor-ethoxy)-benzol (Schmelzpunkt 90°C) und daraus 2-Brom-4,5-diamino-(1,1,2-trifluor-2-chlor)-ethoxybenzol hergestellt.

Beispiel 13 b

(Halogenierung eines Nitranilins und Reduktion)

24 g fein gepulvertes 2-Nitro-4-trifluormethvlmercaptoanilin wurden in 50 ml Trifluoressigsäure gelöst und bei 20°C 18 g Brom zudosiert. Dann wurde für 3 Stunden bei 20°C und für weitere 30 Minuten bei 40°C nachgerührt. Die Mischung auf Wasser gegeben und das Produkt in Dichlormethan aufgenommen. Es fielen nach Entfernung des Lösungsmittels 31 g 6-Brom-2-nitro-4-trifluormethyl-mercapto-anilin an.

155 g des so hergestellten Nitranilins wurden in 700 ml Ethanol mit 15 ml Wasser, 10 ml konzentrierter wäßriger Salzsäure und 70 g Eisenspänen für 15 Stunden zum Sieden am Rückfluß erhitzt, dann das Gemisch abfiltriert, das Filtrat unter reduziertem Druck vom Lösungsmittel befreit und das feste Rohprodukt aus Cyclohexan umkristallisiert. Es wurden 112 g 6-Brom4-trifluormethyl-mercapto-1,2-diaminobenzol mit einem Schmelzpunkt von 60 bis 61°C erhalten.

Beispiel 14 b

Analog Beispiel 13 wurden 27 g 2-Nitro-4-trifluormethyl-sulfonylanilin in 100 ml Essigsäure mit 18 g Brom bromiert. Nach Aufarbeitung fielen 32 g 2-Nitro-6-brom-4-trifluor-methylsulfonyl-anialin an. Schmelzpunkt 147°C.

32 g des so hergestellten Nitramins wurde mit Eisenspänen in Alkohol und wäßriger Chlorwasserstoffsäure reduziert. Es fielen 24 g 3-Brom-5-trifluormethylsulfonyl-phenylen-1,2-diamin an, Schmelzpunkt 155 - 157°C.

Beispiel 15 b

Analog Beispiel 14 wurden 27 g 2-Nitro-4-trifluormethylsulfonyl-anilin in 100 ml Essigsäure mit 10 g Chlor chloriert. Es fielen 29 g 2-Nitro-4-trifluormethylsulfonyl-6-chlor-anilin an, Schmelzpunkt: 138 - 139°C.

Durch Reduktion wurden 13 g 3-Chlor-5-trifluormethylsulfonyl-1,2-phenylendiamin (Schmelzpunkt: 143 - 145°C) erhalten.

Beispiel 16 bis 20 b

(Nitrierung und Reduktion in 2 Stufen)

Beispiel 16 b

263 g 4-(2,6-Dichlor-4-trifluormethyl)-phenoxy-acetanilid wurden in 1 100 ml Dichlormethan gelöst und bei 10°C vorgelegt. Dann wurden bei dieser Temperatur 88 g 98 Gew.-%ige Salpetersäure zugetropft. Es wurde 1 Stunde bei 10°C und 2 weitere Stunden bei 30°C nachgerührt. Nach der Zugabe von 300 ml Wasser wurden die Phasen getrennt und die organische Phase unter reduziertem Druck vom Dichlormethan befreit. Es verblieben 253 g 2-Nitro-4-(2,6-dichlor-4-trifluormethylphenoxy)-acetanilid mit einem Schmelzpunkt von 138 - 140°C.

91 g des so hergestellten Acetanilids wurden in 800 ml Dioxan gelöst, 10 g Raney-Nickel zugegeben und bei 25 bis 45°C in einer Hydrierapparatur mit maximal 50 bar Wasserstoffdruck hydriert. Nach Entspannen und Filtration wurde das Dioxan bei leichtem Vakuum abdestilliert. Es verblieben 65 g 2-Amino-4-(2,6-dichlor-4-trifluormethyl-phenoxy)-acetanilid mit einem Schmelzpunkt von 222 - 223°C.

Beispiel 17 b

Analog zu Beispiel 16 wurde aus 3-Trifluormethyl-4-methoxy-acetanilid zunächst 3-Trifluormethyl-4-methoxy-6-nitro-acetanilid (Schmelzpunkt 143 - 144°C) und daraus 3-Trifluormethyl-4-methoxy-6-amino-acetanilid (Schmelzpunkt 164 - 165°C) hergestellt.

Beispiel 18 b

Analog Beispiel 16 wurde aus 3-Trifluormethyl-4-fluor-trifluormethylacetanilid zunächst 3-Trifluormethyl-4-fluor-6-nitro-trifluormethylacetanilid (Schmelzpunkt 78°C) und daraus 3-Trifluormethyl-4-fluor-6-amino-trifluormethylacetanilid (Schmelzpunkt 92 - 93°C) hergestellt.

Beispiel 19b

Analog Beispiel 16 wurde aus 3-Trifluormethyl-4-brom-trifluormethylacetanilid zunächst 3-Trifluormethyl-4-brom-6-nitro-trifluormethylacetanilid (Schmlezpunkt 110 - 112°C) und daraus 3-Trifluormethyl-4-brom-6-amino-trifluormethylacetanilid (Schmelzpunkt 63 - 65°C) hergestellt.

Beispiel 20 b

Analog Beispiel 16 wurde aus 3-Trifluormethylthio-4-chlor-trifluormethylacetanilid zunächst 3-Trifluormethylthio-4-chlor-6-nitro-trifluormethylacetanilid (Schmelzpunkt 99 - 100°C) und daraus 3-Trifluormethylthio-4-chlor-6-amino-trifluormethylacetanilid (Schmelzpunkt 88 - 90°C) hergestellt.

Beispiel 21 b

0,2 mol 3-Brom-5-trifluormethyl-phenylen-diamin wurden mit 150 ml Trifluoressigsäure für 3 Stunden auf Rückflußtemperatur erhitzt. Zur Aufarbeitung wurde überschüssige Trifluoressigsäure abdestilliert und der Rückstand zwischen 100 ml Wasser und 300 ml Essigester verteilt. Die organische Phase wurde abgetrennt, nacheinander mit jeweils 100 ml wäßriger Natriumhydrogencarbonatlösung und Wasser gewaschen, über Natriumsulfat getrocknet und im Vakuum eingeengt. Der Rückstand wurde durch Chromatographie an Kieselgel (Laufmittel: Cyclohexan/Essigester 1:1) gereinigt.

Man erhielt 4-Brom-6-trifluormethyl-2-trifluormethyl-1H-benzimidazol vom Schmelzpunkt 149 - 151°C.

Beispiel 22 b

0,03 mol 4-Brom-6-trifluormethyl-2-trifluormethyl-1H-benzimidazol und 0,06 mol pulverisiertes Kaliumcarbonat wurden in 70 ml Esigester für 15 Minuten auf Rückflußtemperatur erhitzt, anschließend mit 3,9 g (0,04 mol) Chlormethylmethylthioether in 20 ml Essigester versetzt und unter Rühren für weitere 4 Stunden auf Rückflußtemperatur erhitzt. Zur Aufarbeitung wurde die abgekühlte Reaktionsmischung zweimal mit jeweils 40 ml Wasser gewaschen, über Natriumsulfat getrocknet, im Vakuum eingeengt und der Rückstand durch Chromatographie an Kieselgel (Laufmitel: Dichlormethan) gereinig.

Man erhielt 1-Methylthiomethyl-4-brom-6-trifluormethyl-2-trifluormethyl-benzimidazol vom Schmelzpunkt 56 - 60°C.

**Anwendungsbeispiele:**

In den folgenden Anwendungsbeispielen wurden die nachstehend aufgeführten Verbindungen als Vergleichssubstanzen eingesetzt:

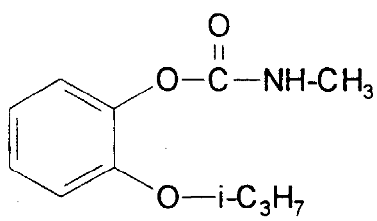

(A)

N-Methyl-O-(2-isopropoxyphenyl)-carbamat (vergl. z.B. DE 11 08 202)

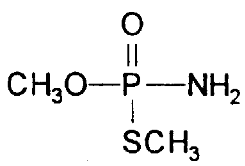

(B)

O,S-Dimethyl-thiolo-phosphorsäureamid (vergl. z.B. DE 12 10 835 )

Beispiel A:

**Phaedon Larven-Test:**

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerettichblattkäfer-Larven (Phaedon cochleariae) besetzt, solange die Blätter noch feucht sind.

Nach der gewünschten Zeit wird die Abtötung in Prozent bestimmt. Dabei bedeutet 100 %, daß alle Käfer-Larven abgetötet wurden; 0 % bedeutet, daß keine Käfer-Larven abgetötet wurden.

Bei diesem Test zeigt z.B. die folgende Verbindung der Herstellungsbeispiele überlegene Wirksamkeit gegenüber dem Stand der Technik: 7.

Tabelle A:

**Phaedon Larven-Test**

| Wirkstoffe | | Wirkstoffkonzentration in % | Abtötungsgrad in % nach 3 Tagen |
|---|---|---|---|
| 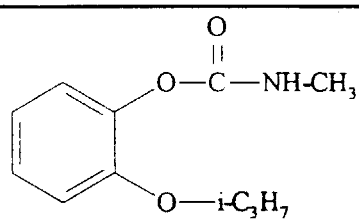 (bekannt) | (A) | 0,1<br>0,01<br>0,001 | 100<br>70<br>0 |
| 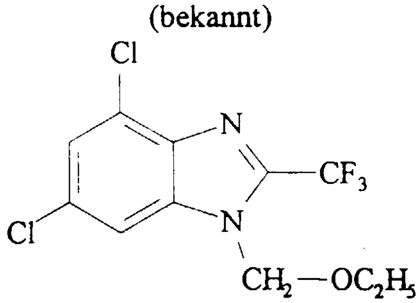 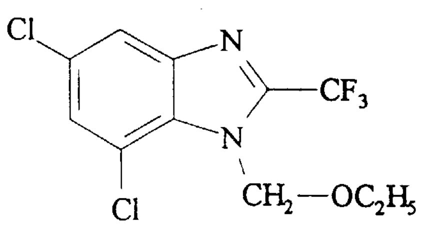 | (7) | 0,1<br>0,01<br>0,001 | 100<br>100<br>100 |

Beispiel B:

**Plutella Test:**

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in Prozent bestimmt. Dabei bedeutet 100 %, daß alle Raupen abgetötet wurden; 0 % bedeutet, daß keine Raupen abgetötet wurden.

Bei diesem Test zeigt z.B. die folgende Verbindung der Herstellungsbeispiele überlegene Wirksamkeit gegenüber dem Stand der Technik: 1 und 7.

## Tabelle B:

**Plutella-Test**

| Wirkstoffe | | Wirkstoffkonzentration in % | Abtötungsgrad in % nach 3 Tagen |
|---|---|---|---|
| 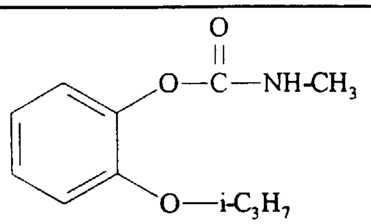 (bekannt) | (A) | 0,1<br>0,01<br>0,001 | 100<br>100<br>10 |
| 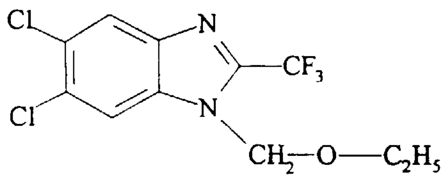 | (1) | 0,1<br>0,01<br>0,001 | 100<br>100<br>100 |
| 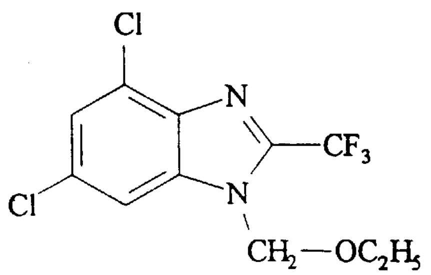 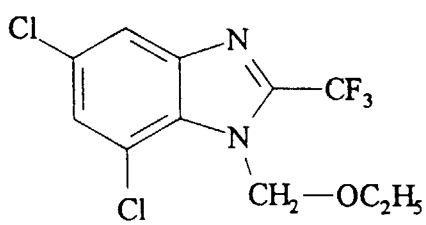 | (7) | 0,1<br>0,01<br>0,001 | 100<br>100<br>100 |

Beispiel C:

**Heliothis virescens-Test**

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) werden durch Tauchen in die Wirkstoffzubereitung der gewünschten Konzentration behandelt und mit der Tabakknospenraupe (Heliothis virescens) besetzt, solange die Blätter noch feucht sind.

Nach der gewünschten Zeit wird die Abtötung in Prozent bestimmt. Dabei bedeutet 100 %, daß alle Raupen abgetötet wurden; 0 % bedeutet, daß keine Raupen abgetötet wurden.

Bei diesem Test zeigt z.B. die folgende Verbindung der Herstellungsbeispiele überlegene Wirksamkeit gegenüber

dem Stand der Technik: 1, 2, 6, 7, 9, 18, 22 und 27.

Tabelle C:

**Heliothis virescens-Test**

| Wirkstoffe | | Wirkstoffkonzentration in % | Abtötungsgrad in % nach 3 Tagen |
|---|---|---|---|
| 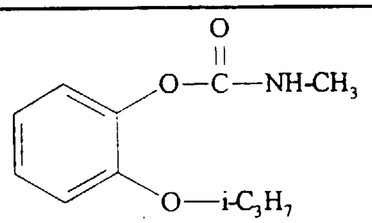 (bekannt) | (A) | 0,1 | 10 |
| 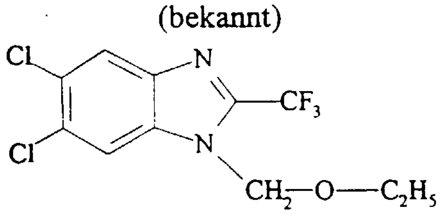 | (1) | 0,1 | 100 |
| 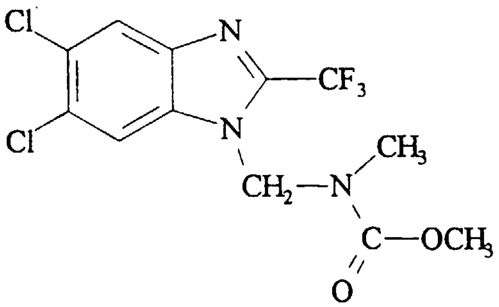 | (2) | 0,1 | 100 |
| 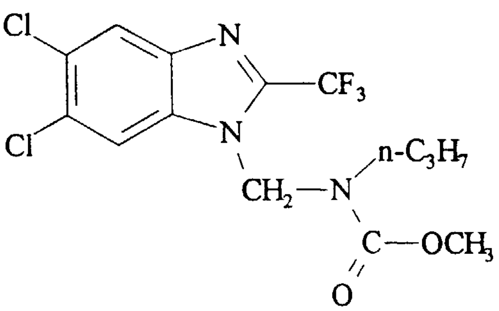 | (6) | 0,1 | 100 |

Tabelle C: (Fortsetzung)

**Heliothis virescens-Test**

| Wirkstoffe | | Wirkstoffkonzentration in % | Abtötungsgrad in % nach 3 Tagen |
|---|---|---|---|
| 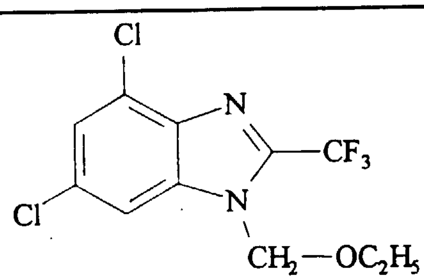 | (7) | 0,1 | 100 |
| 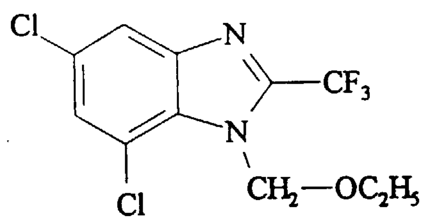 | | | |
| 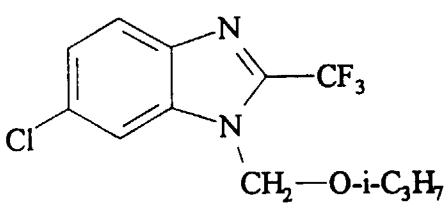 | (9) | 0,1 | 100 |
| 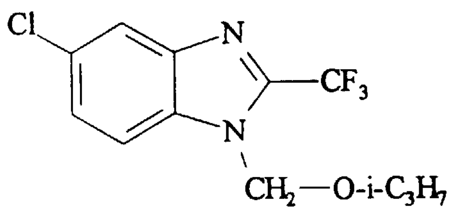 | | | |

Tabelle C: (Fortsetzung)

**Heliothis virescens-Test**

| Wirkstoffe | | Wirkstoffkonzentration in % | Abtötungsgrad in % nach 3 Tagen |
|---|---|---|---|
| 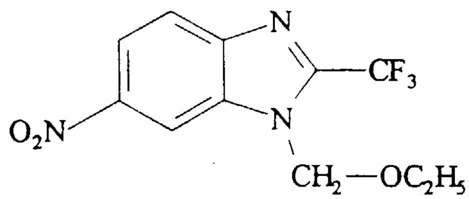 | (18) | 0,1 | 100 |
| 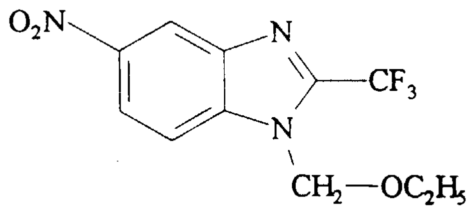 | | | |
| 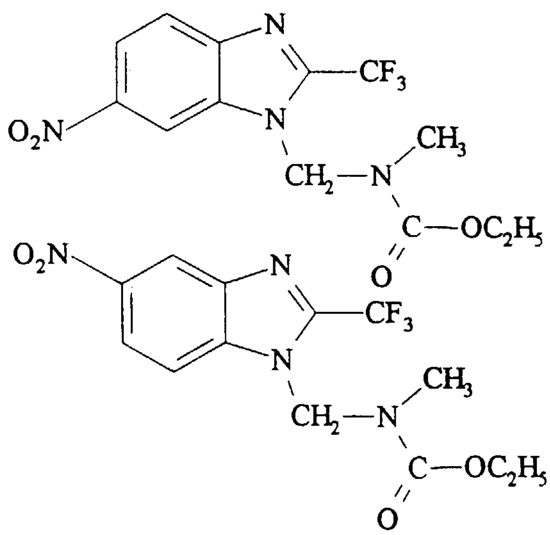 | (22) | 0,1 | 100 |

Tabelle C: (Fortsetzung)

**Heliothis virescens-Test**

| Wirkstoffe | Wirkstoffkonzentration in % | Abtötungsgrad in % nach 3 Tagen |
|---|---|---|
| 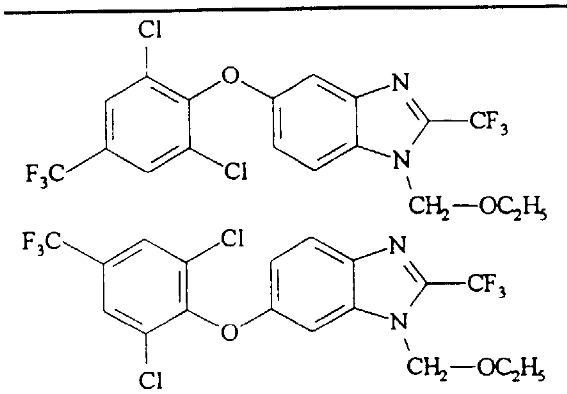 (27) | 0,1 | 100 |

Beispiel D:

**Tetranychus Test (OP-resistent)**

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Entwicklungsstadien der gemeinen Spinnmilbe (Tetranychus urticae) befallen sind, werden in eine Wirkstoffzubereitung der gewünschten Konzentration getaucht.

Nach der gewünschten Zeit wird die Abtötung in Prozent bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 8, 21, 22, 23, 24 und 25.

Tabelle D:

**Tetranychus Test (OP-resistent)**

| Wirkstoffe | | Wirkstoffkonzentration in % | Abtötungsgrad in % nach 7 Tagen |
|---|---|---|---|
| 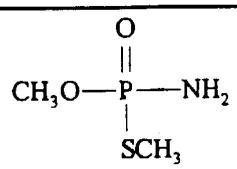 (bekannt) | (B) | 0,01 | 60 |
| 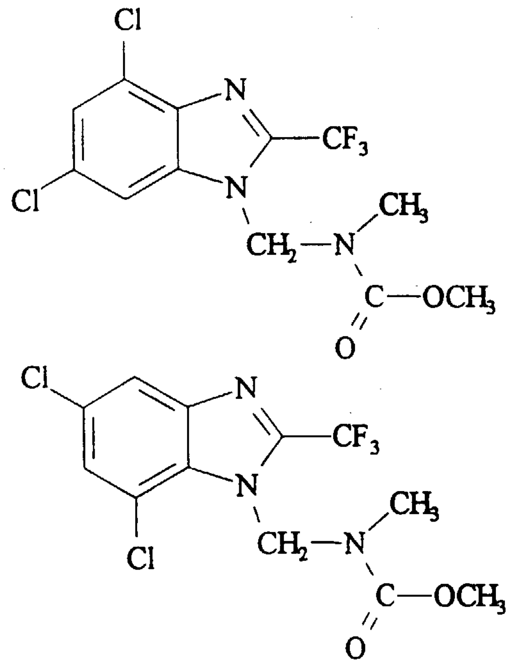 | (8) | 0,01 | 100 |

Tabelle D: (Fortsetzung)

**Tetranychus Test (OP-resistent)**

| **Wirkstoffe** | | **Wirkstoffkonzentration in %** | **Abtötungsgrad in % nach 7 Tagen** |
|---|---|---|---|
| 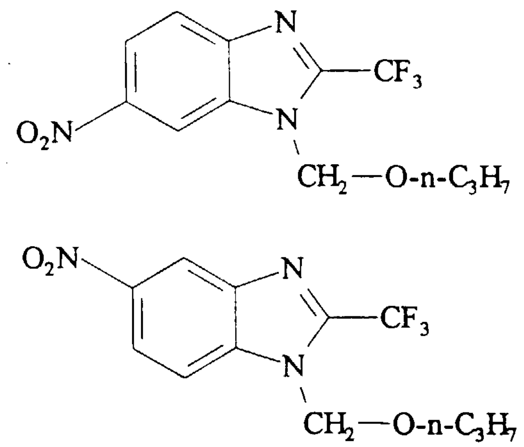 | (21) | 0,01 | 98 |
| 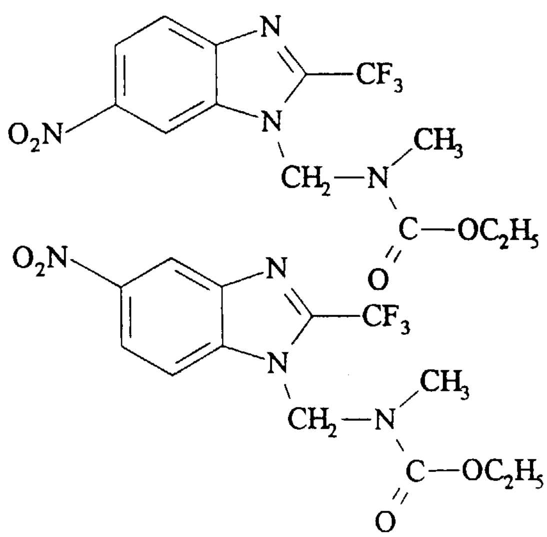 | (22) | 0,01 | 100 |

Tabelle D: (Fortsetzung)

**Tetranychus Test (OP-resistent)**

| Wirkstoffe | Wirkstoff-konzentration in % | Abtötungsgrad in % nach 7 Tagen |
|---|---|---|
| 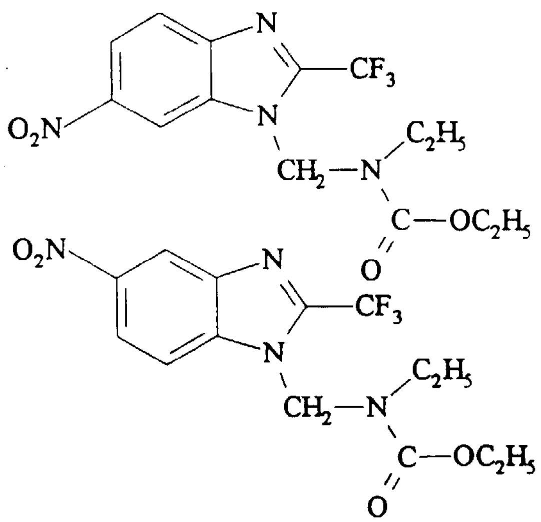 (23) | 0,01 | 100 |
| 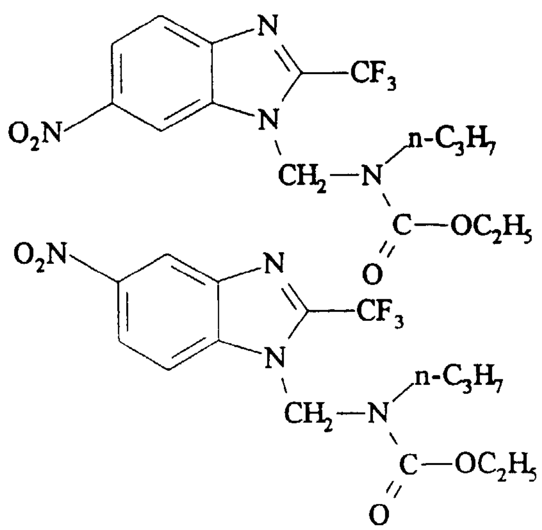 (24) | 0,01 | 100 |

Tabelle D: (Fortsetzung)

**Tetranychus Test (OP-resistent)**

| Wirkstoffe | | Wirkstoff-konzentration in % | Abtötungsgrad in % nach 7 Tagen |
|---|---|---|---|
| 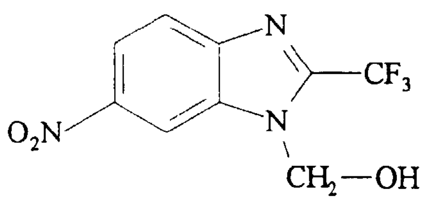 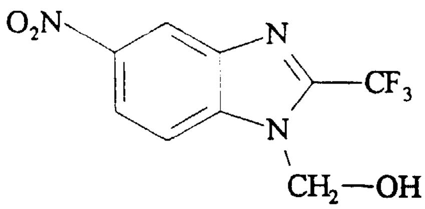 | (25) | 0,01 | 98 |

Beispiel E:

**Nematoden-Grenzkonzentrationstest**

Testnematode: Globodera rostochiensis

Lösungsmittel: 31 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm (= mg/l) angegeben wird. Man füllt den behandelten Boden in Töpfe, pflanzt Kartoffeln ein und hält die Töpfe bei einer Gewächshaustemperatur von 20°C.

Nach sechs Wochen werden die Kartoffelwurzeln auf Zysten untersucht und der Wirkungsgrad des Wirkstoffs in % bestimmt. Der Wirkungsgrad ist 100%, wenn der Befall vollständig vermieden wird, er ist 0%, wenn der Befall genauso hoch ist wie bei den Kontrollpflanzen in unbehandeltem, aber in gleicher Weise verseuchtem Boden.

Bei diesem Test zeigt z.B. die folgende Verbindung der Herstellungsbeispiele überlegene Wirksamkeit gegenüber dem Stand der Technik: 1 und 7.

Tabelle E:

**Nematoden-Test (Globodera rostochiensis)**

| Wirkstoffe | | Abtötungsgrad in % bei einer Wirkstoffkonzentration von 20 ppm |
|---|---|---|
| 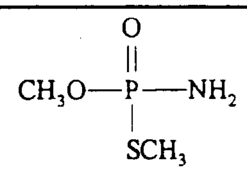 (bekannt) | (B) | 0 |
| 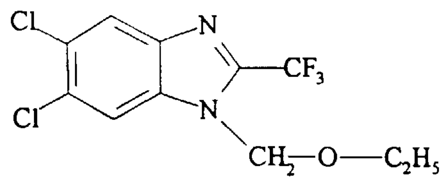 | (1) | 100 |
| 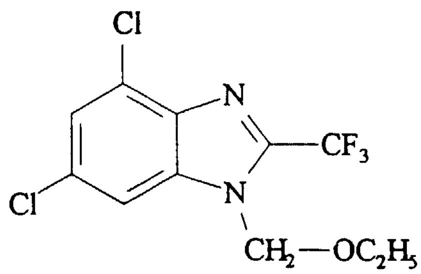 | (7) | 100 |
| 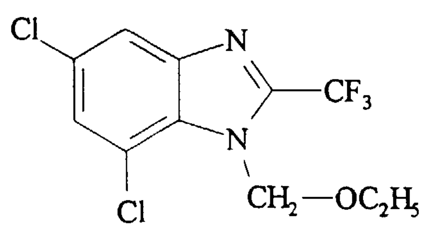 | | |

Beispiel F:

**Psoroptes ovis-Test:**

Lösungsmittel: 35 Gewichtsteile Ethylenglykolmonomethylether

Emulgator: 35 Gewichtsteile Nonylphenolpolyglykolether

Zur Herstellung einer zweckmäßigen Wirkstoffzubereitung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die gewünschte Konzentration.

1 ml dieser Wirkstoffzubereitung wird in PP-Blisterfolien entsprechender Größe pipettiert. Anschließend werden

ca. 25 Milben in die Wirkstoffzubereitung überführt.

Nach 24 Stunden wird die Wirksamkeit der Wirkstoffzubereitung in % bestimmt. 100 % bedeutet, daß alle Milben abgetötet wurden, 0 % bedeutet, daß keine Milben abgetötet wurden.

Bei diesem Test zeigen z.B. die folgenden Verbindungen der Herstellungsbeispiele überlegene Wirksamkeit gegenüber dem Stand der Technik: 3, 4, 5 und 18.

Tabelle F:

**Psoroptes ovis-Test**

| Wirkstoffe | | Wirkstoff-konzentration in ppm a.i. | Abtötungsgrad in % |
|---|---|---|---|
| 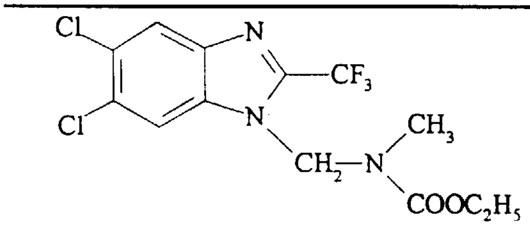 | (3) | 10 | 100 |
| 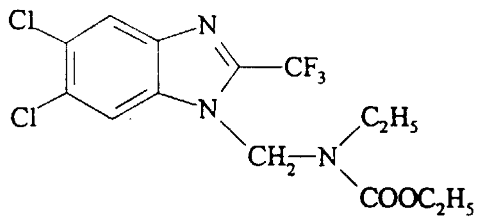 | (4) | 10 | 100 |
| 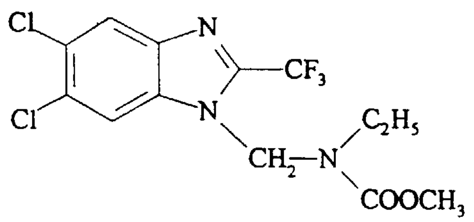 | (5) | 10 | 100 |
| 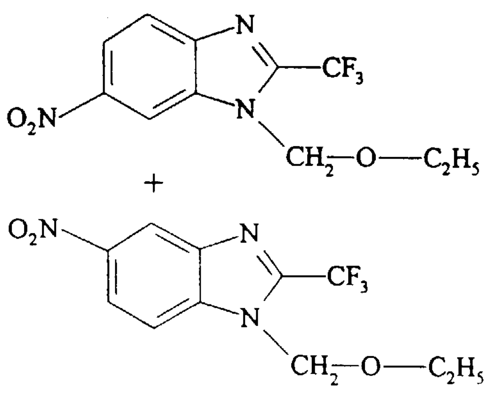 | (18) | 10 | 100 |

Beispiel G:

**Periplaneta americana-Test:**

Lösungsmittel: 35 Gewichtsteile Ethylenglykolmonomethylether

Emulgator: 35 Gewichtsteile Nonylphenolpolyglykolether

Zur Herstellung einer zweckmäßigen Wirkstoffzubereitung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die gewünschte Konzentration.

2 ml dieser Wirkstoffzubereitung wird auf Filterpapierscheiben (Durchmesser: 9,5 cm) pipettiert, die sich in Petrischalen entsprechender Größe befinden. Nach Trocknung der Filterscheiben werden fünf Schaben (Periplaneta americana) in die Petrischalen überführt und abgedeckt.

Nach 3 Tagen wird die Wirksamkeit der Wirkstoffzubereitung in % bestimmt. 100 % bedeutet, daß alle Schaben abgetötet wurden, 0 % bedeutet, daß keine Schaben abgetötet wurden.

Bei diesem Test zeigen z.B. die folgenden Verbindungen der Herstellungsbeispiele überlegene Wirksamkeit gegenüber dem Stand der Technik: 7, 8, 18, 19, 20 und 25.

Tabelle G:

**Periplaneta americana-Test**

| Wirkstoffe | | Wirkstoffkonzentration in ppm a.i. | Abtötungsgrad in % |
|---|---|---|---|
| 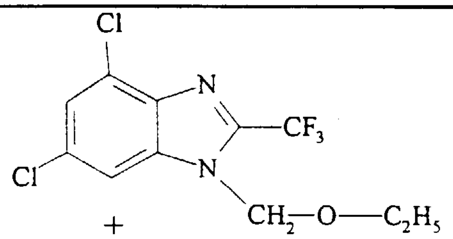 + 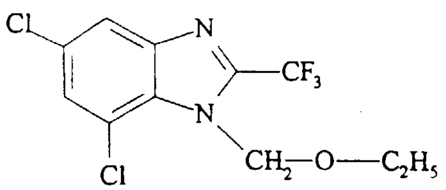 | (7) | 1000<br>100 | 100<br>100 |
| 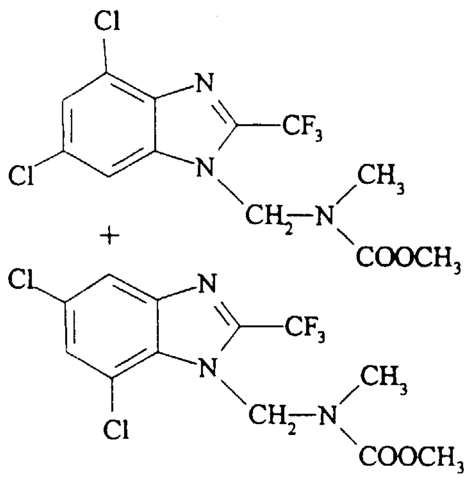 | (8) | 1000<br>100 | 100<br>100 |

Tabelle G: (Fortsetzung)

**Periplaneta americana-Test**

| Wirkstoffe | | Wirkstoff-konzentration in ppm a.i. | Abtötungsgrad in % |
|---|---|---|---|
| 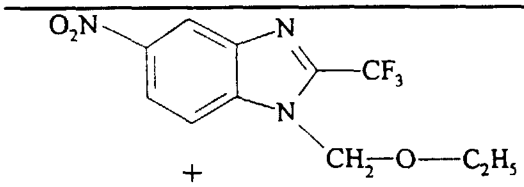 + 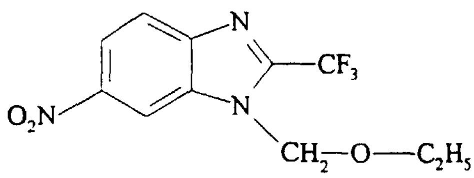 | (18) | 1000 | 100 |
| 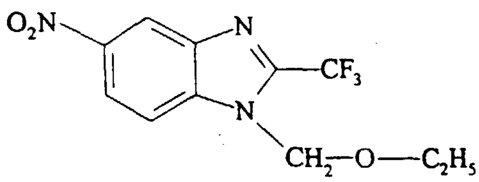 | (19) | 1000 | 100 |
| 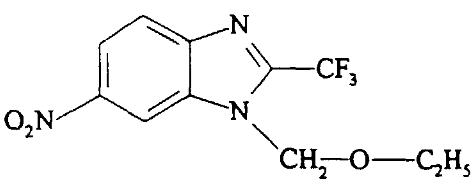 | (20) | 1000 | 100 |
| 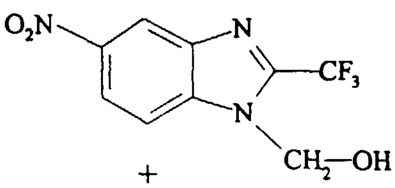 + 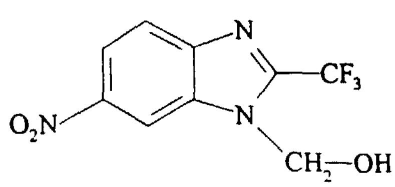 | (25) | 1000 | 100 |

Beispiel H:

**Musca domestica-Test:**

Lösungsmittel: 35 Gewichtsteile Ethylenglykolmonomethylether

Emulgator: 35 Gewichtsteile Nonylphenolpolyglykolether

Zur Herstellung einer zweckmäßigen Wirkstoffzubereitung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die gewünschte Konzentration.

2 ml dieser Wirkstoffzubereitung werden auf Filterpapierscheiben (Durchmesser: 9,5 cm) pipettiert, die sich in Petrischalen entsprechender Größe befinden. Nach Trocknung der Filterscheiben werden 25 Testtiere (Musca domestica; Stamm WHO [N]) in die Petrischalen überführt und abgedeckt.

Nach 3 Tagen wird die Wirksamkeit der Wirkstoffzubereitung in % bestimmt. 100 % bedeutet, daß alle Fliegen abgetötet wurden, 0 % bedeutet, daß keine Fliegen abgetötet wurden.

Bei diesem Test zeigen z.B. die folgenden Verbindungen der Herstellungsbeispiele überlegene Wirksamkeit gegenüber dem Stand der Technik: 7, 11, 18, 19, 20 und 25.

Tabelle H:

**Musca domestica-Test**

| Wirkstoffe | | Wirkstoff-konzentration in ppm a.i. | Abtötungsgrad in % |
|---|---|---|---|
| 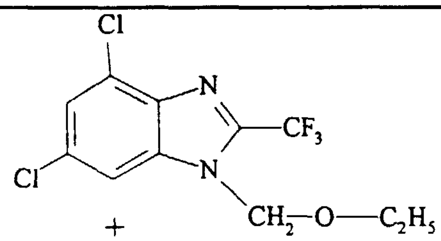 + 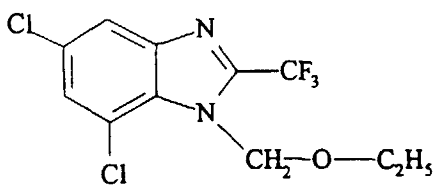 | (7) | 1000<br>100 | 100<br>>50 |
| 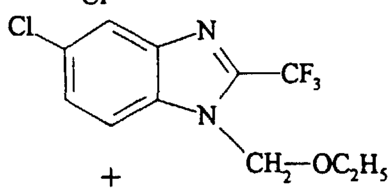 + 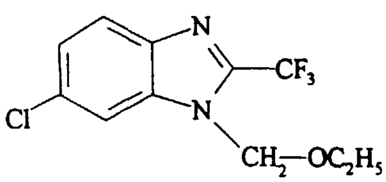 | (11) | 1000 | 100 |

Tabelle H: (Fortsetzung)

**Musca domestica-Test**

| Wirkstoffe | | Wirkstoffkonzentration in ppm a.i. | Abtötungsgrad in % |
|---|---|---|---|
| 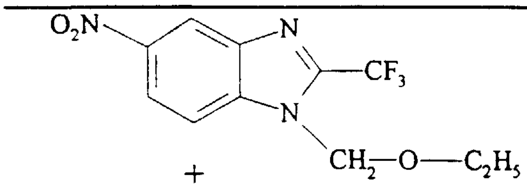<br>+<br>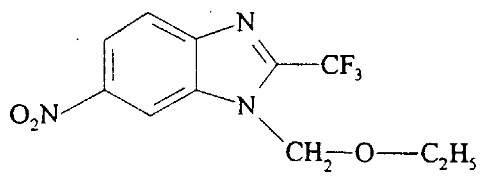 | (18) | 1000<br>100 | 100<br>>50 |
| 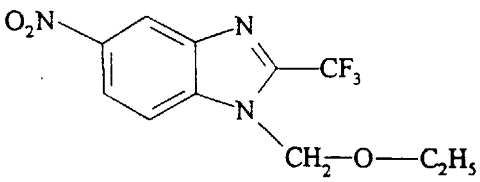 | (19) | 1000<br>100 | 100<br>100 |
| 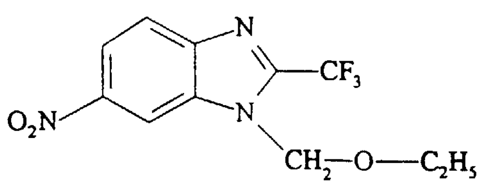 | (20) | 1000<br>100 | 100<br>>50 |
| 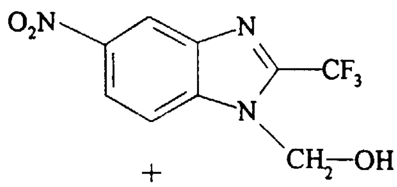<br>+<br>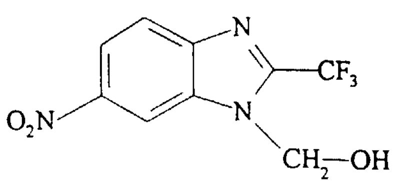 | (25) | 1000 | 100 |

**Patentansprüche**

1. Substituierte Benzimidazole der allgemeinen Formel (I),

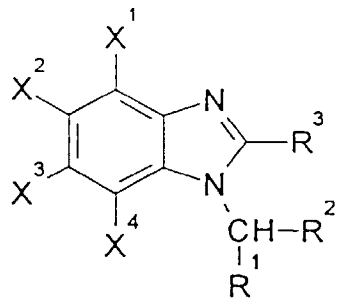

(I)

in welcher

$R^1$ für Wasserstoff, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxyalkyl, Alkoxyalkoxy, Alkanoyl, Alkoxycarbonyl oder Alkoximinoalkyl mit jeweils 1 bis 6 Kohlenstoffatomen in den einzelnen Alkylteilen, gegebenenfalls einfach oder mehrfach, gleich oder verschieden durch Halogen und/oder geradkettiges oder verzweigtes Alkyl mit 1 bis 6 Kohlenstoffatomen und/oder geradkettiges oder verzweigtes Halogenalkyl mit 1 bis 6 Kohlenstoffatomen und 1 bis 13 gleichen oder verschiedenen Halogenatomen substituiertes, zweifach verknüpftes Dioxyalkylen mit 1 bis 5 Kohlenstoffatomen oder gegebenenfalls einfach oder mehrfach, gleich oder verschieden durch Halogen und/oder geradkettiges oderverzweigtes Alkyl mit 1 bis 6 Kohlenstoffatomen und/oder geradkettiges oder verzweigtes Halogenalkyl mit 1 bis 6 Kohlenstoffatomen und 1 bis 13 gleichen oder verschiedenen Halogenatomen substituiertes Phenyl,

$R^2$ für Hydroxy, Cyano, Alkoxy mit 1 bis 8 Kohlenstoffatomen oder für gegebenenfalls einfach oder zweifach, gleich oder verschieden substituiertes Amino steht, wobei als Substituenten infrage kommen:
geradkettiges oder verzweigtes Alkyl mit 1 bis 8 Kohlenstoffatomen, geradkettiges oder verzweigtes Alkenyl mit 2 bis 8 Kohlenstoffatomen, Cycloalkyl mit 3 bis 8 Kohlenstoffatomen, Alkoxycarbonyl, Alkylthio-carbonyl, Alkoxy-thiocarbonyl oder Alkylthio-thiocarbonyl mit jeweils 1 bis 8 Kohlenstoffatomen in den einzelnen geradkettigen oder verzweigten Alkylteilen, zweifach verknüpftes, ringgeschlossenes Alkandiyloxycarbonyl mit 2 bis 6 Kohlenstoffatomen im Alkandiylteil oder jeweils gegebenenfalls einfach oder mehrfach, gleich oder verschieden substituiertes Phenyl oder Phenylalkyl mit 1 bis 6 Kohlenstoffatomen im geradkettigen oder verzweigten Alkylteil, wobei als Substituenten die bei $R^1$ genannten Substituenten infrage kommen.

$R^3$ für geradkettiges oder verzweigtes Perhalogenalkyl mit 1 bis 8 Kohlenstoffatomen und 1 bis 17 gleichen oder verschiedenen Halogenatomen steht und

$X^1$, $X^2$, $X^3$ und $X^4$ unabhängig voneinander jeweils für Wasserstoff, Fluor, Chlor, Brom, Iod, Nitro oder für gegebenenfalls einfach oder mehrfach, gleich oder verschieden substituiertes Phenoxy stehen,wobei als Substituenten die bei $R^1$ genannten infrage kommen, wobei jedoch mindestens einer der Substituenten $X^1$, $X^2$, $X^3$ oder $X^4$ verschieden von Wasserstoff ist und

wobei die Verbindung 1-Cyanomethyl-2-trifluormethyl-5,6-dichlorbenzimidazol ausgenommen ist.

**2.** Verbindungen der Formel (I) gemäß Anspruch 1, bei welchen

$R^1$ für Wasserstoff oder für geradkettiges oder verzweigtes Alkyl mit 1 bis 6 Kohlenstoffatomen steht,

$R^2$ für Hydroxy, Cyano, Alkoxy mit 1 bis 6 Kohlenstoffatomen oder für gegebenenfalls einfach oder zweifach, gleich oder verschieden substituiertes Amino steht, wobei als Substituenten infrage kommen:
geradkettiges oder verzweigtes Alkyl mit 1 bis 6 Kohlenstoffatomen, geradkettiges oder verzweigtes Alkenyl mit 2 bis 6 Kohlenstoffatomen, Cycloalkyl mit 3 bis 7 Kohlenstoffatomen, Alkoxycarbonyl, Alkylthio-carbonyl, Alkoxy-thiocarbonyl oder Alkylthio-thiocarbonyl mit jeweils 1 bis 6 Kohlenstoffatomen in den einzelnen geradkettigen oder verzweigten Alkylteilen.

$R^3$ für geradkettiges oder verzweigtes Perhalogenalkyl mit 1 bis 6 Kohlenstoffatomen und 1 bis 13 gleichen oder verschiedenen Halogenatomen steht und

$X^1$, $X^2$, $X^3$ und $X^4$ unabhängig voneinander jeweils für Wasserstoff, Fluor, Chlor, Brom, Iod, Nitro oder für gegebenenfalls einfach bis fünffach, gleich oder verschieden substituiertes Phenoxy stehen, wobei als Substituenten die folgenden infrage kommen
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxyalkyl, Alkoxyalkoxy, Alkanoyl, Alkoxycarbonyl oder Alkoximinoalkyl mit jeweils 1 bis 4 Kohlenstoffatomen in den einzelnen Alkylteilen, gegebenenfalls einfach bis sechsfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, zweifach verknüpftes Dioxyalkylen mit 1 bis 4 Kohlenstoffatomen oder gegebenenfalls einfach bis fünffach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes Phenyl, wobei jedoch mindestens einer der Substituenten $X^1$, $X^2$, $X^3$ oder $X^4$ verschieden von Wasserstoff ist und

wobei die Verbindung 1-Cyanomethyl-2-trifluormethyl-5,6-dichlorbenzimidazol ausgenommen ist.

**3.** Verbindungen der Formel (I) gemäß Anspruch 1, bei welchen

$R^1$ für Wasserstoff, für geradkettiges oder verzweigtes Alkyl mit1 bis 4 Kohlenstoffatomen steht,

$R^2$ für Hydroxy, Cyano, Alkoxy mit 1 bis 6 Kohlenstoffatomen oder für gegebenenfalls einfach oder zweifach, gleich oder verschieden substituiertes Amino steht, wobei als Substituenten infrage kommen:
geradkettiges oder verzweigtes Alkyl mit 1 bis 4 Kohlenstoffatomen, geradkettiges oder verzweigtes Alkenyl mit 2 bis 4 Kohlenstoffatomen, Cycloalkyl mit 3 bis 6 Kohlenstoffatomen, Alkoxycarbonyl, Alkylthio-carbonyl, Alkoxy-thiocarbonyl oder Alkylthio-thiocarbonyl mit jeweils 1 bis 4 Kohlenstoffatomen in den einzelnen geradkettigen oder verzweigten Alkylteilen,

$R^3$ für geradkettiges oder verzweigtes Perhalogenalkyl mit 1 bis 4 Kohlenstoffatomen und 1 bis 9 gleichen oder verschiedenen Halogenatomen steht,

$X^1$, $X^2$, $X^3$ und $X^4$ unabhängig voneinander jeweils für Wasserstoff, Chlor, Brom, Nitro oder für gegebenenfalls im Phenylteil einfach bis dreifach, gleich oder verschieden substituiertes Phenyloxy stehen, wobei als Phenylsubstituenten jeweils die folgenden infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen,
wobei jedoch mindestens einer der Substituenten $X^1$,$X^2$,$X^3$ oder $X^4$ verschieden von Wasserstoff ist und

wobei die Verbindung 1-Cyanomethyl-2-trifluormethyl-5,6-dichlorbenzimidazol ausgenommen ist.

**4.** Verfahren zur Herstellung der substituierten Benzimidazole der allgemeinen Formel (I) gemäß Anspruch 1

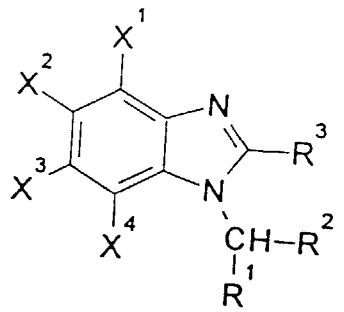

(I)

in welcher die Substituenten $R^1$ - $R^3$ und $X^1$- $X^4$ die in Anspruch 1 angegebene Bedeutung haben dadurch gekennzeichnet, daß man 1H-Benzimidazole der Formel (II),

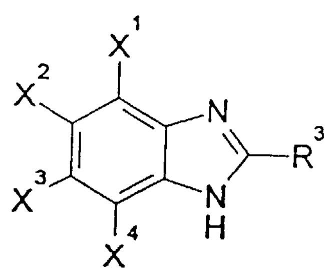

in welcher
$R^3$,$X^1$, $X^2$, $X^3$ und $X^4$ die oben angegebene Bedeutung haben,
mit Verbindungen der Formel (III),

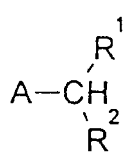

(III)

in welcher

A für eine geeignete Abgangsgruppe steht,

$R^1$ die oben angegebene Bedeutung hat und

$R^2$ die oben angegebene Bedeutung hat

gegebenenfalls in Gegenwart eines Verdünnungsmittels und gegebenenfalls in Gegenwart eines Reaktionshilfsmittels umsetzt.

**5.** Schädlingsbekämpfungsmittel, gekennzeichnet durch einen Gehalt an mindestens einem substituierten Benzimidazol der Formel (I), gemäß Anspruch 1.

**6.** Verwendung von substituierten Benzimidazolen der Formel (I) gemäß Anspruch 1 zur Bekämpfung von Schädlingen.

**7.** Verfahren zur Herstellung von Schädlingsbekämpfungsmitteln, dadurch gekennzeichnet, daß man substituierte Benzimidazole der Formel (I) gemäß Anspruch 1 mit Streckmitteln und/oder oberflächenaktiven Mitteln vermischt.

**Claims**

**1.** Substituted benzimidazoles of the general formula (I)

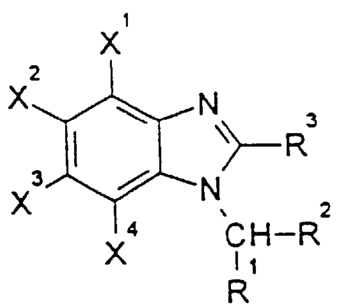

(I)

in which

$R^1$ represents hydrogen, represents straight-chain or branched alkyl having 1 to 8 carbon atoms or represents phenyl which is optionally substituted one or more times by identical or different substituents, suitable substituents being:
halogen, cyano, nitro, in each case straight-chain or branched alkyl, alkoxy, alkylthio, alkylsulphinyl or alkylsulphonyl having in each case 1 to 6 carbon atoms, in each case straight-chain or branched halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms, in each case straight-chain or branched alkoxyalkyl, alkoxyalkoxy, alkanoyl, alkylcarbonyl or alkoximinoalkyl having in each case 1 to 6 carbon atoms in the individual alkyl moieties, divalent dioxyalkylene having 1 to 5 carbon atoms which is optionally substituted one or more times by identical or different substituents consisting of halogen and/or straight-chain or branched alkyl having 1 to 6 carbon atoms and/or straight-chain or branched halogenoalkyl having 1 to 6 carbon atoms and 1 to 13 identical or different halogen atoms, or phenyl which is optionally substituted one or more times by identical or different substituents consisting of halogen and/or straight-chain or branched alkyl having 1 to 6 carbon atoms and/or straight-chain or branched halogenoalkyl having 1 to 6 carbon atoms and 1 to 13 identical or different halogen atoms,

$R^2$ represents hydroxyl, cyano, alkoxy having 1 to 8 carbon atoms or represents amino which is optionally substituted one or two times by identical or different substituents, suitable substituents being:
straight-chain or branched alkyl having 1 to 8 carbon atoms, straight-chain or branched alkenyl having 2 to 8 carbon atoms, cycloalkyl having 3 to 8 carbon atoms, alkoxycarbonyl, alkylthiocarbonyl, alkoxy-thiocarbonyl or alkylthio-thiocarbonyl having in each case 1 to 8 carbon atoms in the individual straight-chain or branched alkyl moieties, divalent, ring-closed alkanediyloxycarbonyl having 2 to 6 carbon atoms in the alkanediyl moiety, or phenyl or phenylalkyl having 1 to 6 carbon atoms in the straight-chain or branched alkyl moiety, each of which is optionally substituted one or more times by identical or different substituents, suitable substituents being the substituents specified in the case of $R^1$,

$R^3$ represents straight-chain or branched perhalogenoalkyl having 1 to 8 carbon atoms and

1 to 17 identical or different halogen atoms, and

$X^1$, $X^2$, $X^3$ and $X^4$ independently of one another in each case represent hydrogen, fluorine, chlorine, bromine, iodine, nitro or represent phenoxy which is optionally substituted one or more times by identical or different substituents, suitable substituents being those specified in the case of $R^1$, but where at least one of the substituents $X^1$, $X^2$, $X^3$ or $X^4$ is other than hydrogen, and

with the exception of the compound 1-cyanomethyl-2-trifluoromethyl-5,6-dichlorobenzimidazole.

**2.** Compounds of the formula (I) according to Claim 1, in which

$R^1$ represents hydrogen or represents straight-chain or branched alkyl having 1 to 6 carbon atoms,

$R^2$ represents hydroxyl, cyano, alkoxy having 1 to 6 carbon atoms or represents amino which is optionally substituted one or two times by identical or different substituents, suitable substituents being:
straight-chain or branched alkyl having 1 to 6 carbon atoms, straight-chain or branched alkenyl having 2 to 6 carbon atoms, cycloalkyl having 3 to 7 carbon atoms, alkoxycarbonyl, alkylthiocarbonyl, alkoxy-thiocarbonyl or alkylthio-thiocarbonyl having in each case 1 to 6 carbon atoms in the individual straight-chain or branched alkyl moieties,

$R^3$ represents straight-chain or branched perhalogenoalkyl having 1 to 6 carbon atoms and 1 to 13 identical or different halogen atoms, and

$X^1$, $X^2$, $X^3$ and $X^4$ independently of one another in each case represent hydrogen, fluorine, chlorine, bromine, iodine, nitro or represent phenoxy which is optionally substituted one to five times by identical or different substituents, suitable substituents being the following:
halogen, cyano, nitro, in each case straight-chain or branched alkyl, alkoxy, alkylthio, alkylsulphinyl or alkylsulphonyl having in each case 1 to 4 carbon atoms, in each case straight-chain or branched halogenoalkyl, halogenoalkoxy, halogenoalkylthio, halogenoalkylsulphinyl or halogenoalkylsulphonyl having in each case 1 to 4 carbon atoms and 1 to 9 identical or different halogen atoms, in each case straight-chain or branched alkoxyalkyl, alkoxyalkoxy, alkanoyl, alkylcarbonyl or alkoximinoalkyl having in each case 1 to 4 carbon atoms in the individual alkyl moieties, divalent dioxyalkylene having 1 to 4 carbon atoms which is optionally substituted one to six times by identical or different substituents consisting of halogen and/or straight-chain or branched alkyl having 1 to 4 carbon atoms and/or straight-chain or branched halogenoalkyl having 1 to 4 carbon atoms and 1 to 9 identical or different halogen atoms, or phenyl which is optionally substituted one to five times by identical or different substituents consisting of halogen and/or straight-chain or branched alkyl having 1 to 4 carbon atoms and/or straight-chain or branched halogenoalkyl having I to 4 carbon atoms and 1 to 9 identical or different halogen atoms, but where at least one of the substituents $X^1$, $X^2$, $X^3$ or $X^4$ is other than hydrogen, and

with the exception of the compound 1-cyanomethyl-2-trifluoromethyl-5,6-dichlorobenzimidazole.

**3.** Compounds of the formula (I) according to Claim 1, in which

$R^1$ represents hydrogen, represents straight-chain or branched alkyl having 1 to 4 carbon atoms,

$R^2$ represents hydroxyl, cyano, alkoxy having 1 to 6 carbon atoms or represents amino which is optionally substituted one or two times by identical or different substituents, suitable substituents being:
straight-chain or branched alkyl having 1 to 4 carbon atoms, straight-chain or branched alkenyl having 2 to 4 carbon atoms, cycloalkyl having 3 to 6 carbon atoms, alkoxycarbonyl, alkylthiocarbonyl, alkoxy-thiocarbonyl or alkylthio-thiocarbonyl having in each case 1 to 4 carbon atoms in the individual straight-chain or branched alkyl moieties,

$R^3$ represents straight-chain or branched perhalogenoalkyl having I to 4 carbon atoms and 1 to 9 identical or different halogen atoms, and

$X^1$, $X^2$, $X^3$ and $X^4$ independently of one another in each case represent hydrogen, chlorine, bromine, nitro or represents phenyloxy which is optionally substituted in the phenyl moiety one to three times by identical or different substituents, suitable substituents for phenyl being in each case the following:
halogen, cyano, nitro, in each case straight-chain or branched alkyl, alkoxy, alkylthio, alkylsulphinyl or alkylsulphonyl having in each case 1 to 3 carbon atoms, in each case straight-chain or branched halogenoalkyl, halogenoalkoxy, halogenoalkylthio, halogenoalkylsulphinyl or halogenoalkylsulphonyl having in each case 1 to 3 carbon atoms and 1 to 7 identical or different halogen atoms,
but where at least one of the substituents $X^1$, $X^2$, $X^3$ or $X^4$ is other than hydrogen, and

with the exception of the compound 1-cyanomethyl-2-trifluoromethyl-5,6-dichlorobenzimidazole.

**4.** Process for the preparation of the substituted benzimidazoles of the general formula (I) according to Claim 1

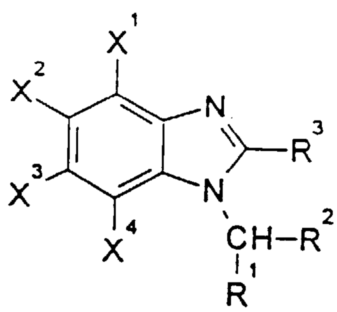

(I)

in which the substituents $R^1$-$R^3$ and $X^1$-$X^4$ have the meaning given in Claim 1
characterized in that 1H-benzimidazoles of the formula (II)

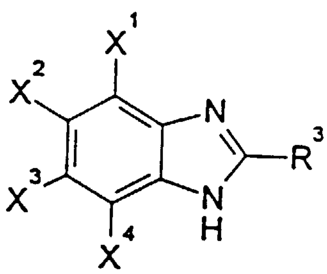

in which
$R^3$, $X^1$, $X^2$, $X^3$ and $X^4$ have the meaning given above,
are reacted with compounds of the formula (III)

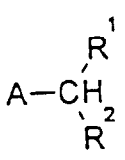

(III)

in which

A represents a suitable leaving group,

$R^1$ has the meaning given above and

$R^2$ has the meaning given above

optionally in the presence of a diluent and optionally in the presence of a reaction auxiliary.

**5.** Compositions for combating pests, characterized by a content of at least one substituted benzimidazole of the formula (I) according to Claim 1.

**6.** Use of substituted benzimidazoles of the formula (I) according to Claim 1 for combating pests.

**7.** Compositions for the preparation of agents for combating pests, characterized in that substituted benzimidazoles of the formula (I) according to Claim 1 are mixed with extenders and/or surface-active agents.

**Revendications**

**1.** Benzimidazoles substitués de formule générale (I)

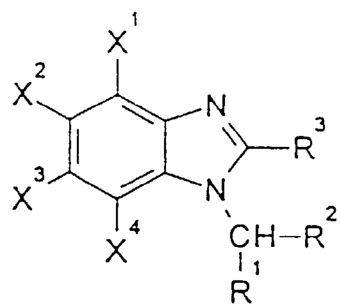

(I)

dans laquelle

$R^1$ représente de l'hydrogène, un groupe alkyle linéaire ou ramifié ayant 1 à 8 atomes de carbone ou un groupe phényle portant, le cas échéant, un ou plusieurs substituants identiques ou différents, avec comme substituants :
un halogène, un groupe cyano, nitro, un groupe linéaire ou ramifié alkyle, alkoxy, alkylthio, alkylsulfinyle ou alkylsulfonyle ayant, dans chaque cas, 1 à 6 atomes de carbone, un groupe linéaire ou ramifié halogénalkyle, halogénalkoxy, halogénalkylthio, halogénalkylsulfinyle ou halogénalkylsulfonyle ayant, dans chaque cas, 1 à 6 atomes de carbone et 1 à 13 atomes d'halogènes identiques ou différents, un groupe linéaire ou ramifié alkoxyalkyle, alkoxyalkoxy, alcanoyle, alkoxycarbonyle ou alkoximinoalkyle ayant, dans chaque cas, 1 à 6 atomes de carbone dans les parties alkyle individuelles, un groupe dioxyalkylène divalent ayant 1 à 5 atomes de carbone, substitué, le cas échéant, une ou plusieurs fois identiques ou différentes par un halogène et/ou un radical alkyle linéaire ou ramifié ayant 1 à 6 atomes de carbone et/ou un radical halogénalkyle linéaire ou ramifié ayant 1 à 6 atomes de carbone et 1 à 13 atomes d'halogènes identiques ou différents, ou un groupe phényle substitué, le cas échéant, une ou plusieurs fois identiques ou différentes par un halogène et/ou un radical alkyle linéaire ou ramifié ayant 1 à 6 atomes de carbone et/ou un radical halogénalkyle linéaire ou ramifié ayant 1 à 6 atomes de carbone et 1 à 13 atomes d'halogènes identiques ou différents,

$R^2$ est un groupe hydroxy, cyano, un groupe alkoxy ayant 1 à 8 atomes de carbone ou un groupe amino éventuellement substitué une ou deux fois identiques ou différentes, en considérant comme substituants :
un groupe alkyle linéaire ou ramifié ayant 1 à 8 atomes de carbone, un groupe alcényle linéaire ou ramifié ayant 2 à 8 atomes de carbone, un groupe cycloalkyle de 3 à 8 atomes de carbone, un groupe alkoxycarbonyle, alkylthiocarbonyle, alkoxythiocarbonyle ou alkylthio-thiocarbonyle ayant, dans chaque cas, 1 à 8 atomes de carbone dans les parties alkyle

individuelles linéaires ou ramifiées, un groupe alcanediyloxycarbonyle cyclisé divalent ayant 2 à 6 atomes de carbone dans la partie alcanediyle ou un groupe phényle ou un groupe phénylalkyle de 1 à 6 atomes de carbone dans la partie alkyle linéaire ou ramifiée, portant chacun, le cas échéant, un ou plusieurs substituants identiques ou différents, en considérant comme substituants les substituants mentionnés dans le cas de $R^1$,

$R^3$ est un groupe perhalogénalkyle linéaire ou ramifié ayant 1 à 8 atomes de carbone et 1 à 17 atomes d'halogènes identiques ou différents et

$X^1$, $X^2$, $X^3$ et $X^4$ représentent chacun, indépendamment les uns des autres, de l'hydrogène, du fluor, du chlore, du brome, de l'iode, un groupe nitro ou un groupe phénoxy portant, le cas échéant, un ou plusieurs substituants identiques ou différents, en considérant comme substituants ceux qui sont mentionnés dans le cas de $R^1$, mais au moins l'un des substituants $X^1$, $X^2$, $X^3$ ou $X^4$ étant différent de l'hydrogène, et

le composé 1-cyanométhyl-2-trifluorométhyl-5,6-dichlorobenzimidazole étant exclu.

**2.** Composés de formule (I) suivant la revendication 1, dans lesquels

$D^1$ représente de l'hydrogène ou un groupe alkyle linéaire ou ramifié ayant 1 à 6 atomes de carbone,

$R^2$ est un groupe hydroxy, cyano, un groupe alkoxy ayant 1 à 6 atomes de carbone ou un groupe amino portant, le cas échéant, un ou deux substituants identiques ou différents, les substituants considérés étant :
un groupe alkyle linéaire ou ramifié ayant 1 à 6 atomes de carbone, un groupe alcényle linéaire ou ramifié ayant 2 à 6 atomes de carbone, un groupe cycloalkyle de 3 à 7 atomes de carbone, un groupe alkoxycarbonyle, alkylthiocarbonyle, alkoxythiocarbonyle ou alkylthio-thiocarbonyle ayant chacun 1 à 6 atomes de carbone dans les parties alkyle individuelles linéaires ou ramifiées,

$R^3$ est un groupe perhalogénalkyle linéaire ou ramifié ayant 1 à 6 atomes de carbone et 1 à 13 atomes d'halogènes identiques ou différents et

$X^1$, $X^2$, $X^3$ et $X^4$ et $X^4$ représentent chacun, indépendamment les uns des autres, de l'hydrogène, du fluor, du chlore, du brome, de l'iode, un groupe nitro ou un groupe phénoxy portant, le cas échéant, un à cinq substituants identiques ou différents, en considérant comme substituants les substituants suivants :
un halogène, un groupe cyano, nitro, un groupe linéaire ou ramifié alkyle, alkoxy, alkylthio, alkylsulfinyle ou alkylsulfonyle ayant chacun 1 à 4 atomes de carbone, un groupe linéaire ou ramifié halogénalkyle, halogénalkoxy, halogénalkylthio, halogénalkylsulfinyle ou halogénalkylsulfonyle ayant chacun 1 à 4 atomes de carbone et 1 à 9 atomes d'halogènes identiques ou différents, un groupe linéaire ou ramifié alkoxyalkyle, alkoxyalkoxy, alcanoyle, alkoxycarbonyle ou alkoximinoalkyle ayant chacun 1 à 4 atomes de carbone dans les parties alkyle individuelles, un groupe dioxyalkylène divalent ayant 1 à 4 atomes de carbone, éventuellement substitué une à six fois identiques ou différentes par un halogène et/ou un groupe alkyle linéaire ou ramifié ayant 1 à 4 atomes de carbone et/ou un groupe halogénalkyle linéaire ou ramifié ayant 1 à 4 atomes de carbone et 1 à 9 atomes d'halogènes identiques ou différents, ou un groupe phényle éventuellement substitué une à cinq fois identiques ou différentes par un halogène et/ou un groupe alkyle linéaire ou ramifié ayant 1 à 4 atomes de carbone et/ou un groupe halogénalkyle linéaire ou ramifié ayant 1 à 4 atomes de carbone et 1 à 9 atomes d'halogènes identiques ou différents, mais au moins l'un des substituants $X^1$, $X^2$, $X^3$ ou $X^4$ étant différent de l'hydrogène, et

le composé 1-cyanométhyl-2-trifluorométhyl-5,6-dichlorobenzimidazole étant exclu.

**3.** Composés de formule (I) suivant la revendication 1, dans lesquels

$R^1$ est de l'hydrogène, un groupe alkyle linéaire ou ramifié ayant 1 à 4 atomes de carbone,

$R^2$ est un groupe hydroxy, cyano, un groupe alkoxy ayant 1 à 6 atomes de carbone ou un groupe amino portant, le cas échéant, un ou deux substituants identiques ou différents, en considérant comme substituants :
un groupe alkyle linéaire ou ramifié ayant 1 à 4 atomes de carbone, un groupe alcényle

linéaire ou ramifié ayant 2 à 4 atomes de carbone, un groupe cycloalkyle de 3 à 6 atomes de carbone, un groupe alkoxycarbonyle, alkylthiocarbonyle, alkoxythiocarbonyle ou alkylthio-thiocarbonyle ayant, dans chaque cas, 1 à 4 atomes de carbone dans les parties alkyle individuelles linéaires ou ramifiées,

$R^3$ est un groupe perhalogénalkyle linéaire ou ramifié ayant 1 à 4 atomes de carbone et 1 à 9 atomes d'halogènes identiques ou différents, et

$X^1$, $X^2$, $X^3$ et $X^4$ représentent chacun, indépendamment les uns des autres, de l'hydrogène, du chlore, du brome, un groupe nitro ou un groupe phényloxy portant éventuellement dans la partie phényle un à trois substituants identiques ou différents, en considérant comme substituants du groupe phényle chacun des substituants suivants :
un halogène, un groupe cyano, un groupe nitro, un groupe linéaire ou ramifié alkyle, alkoxy, alkylthio, alkylsulfinyle ou alkylsulfonyle ayant, dans chaque cas, 1 à 3 atomes de carbone, un groupe linéaire ou ramifié halogénalkyle, halogénalkoxy, halogénalkylthio, halogénalkylsulfinyle ou halogénalkylsulfonyle ayant, dans chaque cas, 1 à 3 atomes de carbone et 1 à 7 atomes d'halogènes identiques ou différents, mais au moins l'un des substituants $X^1$, $X^2$, $X^3$ ou $X^4$ étant différent de l'hydrogène, et

le composé 1-cyanométhyl-2-trifluorométhyl-5,6-dichlorobenzimidazole étant exclu.

**4.** Procédé de production des benzimidazoles substitués de formule générale (I) suivant la revendication 1

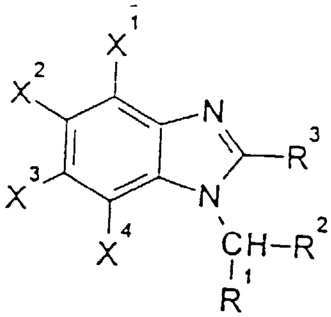

(I)

dans laquelle les substituants $R^1$ à $R^3$ et $X^1$ à $X^4$ ont la définition indiquée dans la revendication 1,
caractérisé en ce qu'on fait réagir des 1H-benzimidazoles de formule (II)

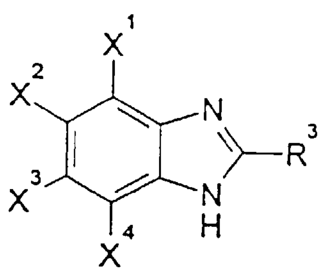

dans laquelle
$R^3$, $X^1$, $X^2$, $X^3$ et $X^4$ ont la définition indiquée ci-dessus,
avec des composés de formule (III)

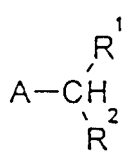

(III)

dans laquelle

A est un groupe partant approprié,
$R^1$ a la définition indiquée ci-dessus et
$R^2$ a la définition indiquée ci-dessus,

le cas échéant en présence d'un diluant et en la présence éventuelle d'un auxiliaire de réaction.

**5.** Composition pesticide, caractérisée par une teneur en au moins un benzimidazole substitué de formule (I) suivant la revendication 1.

**6.** Utilisation de benzimidazoles substitués de formule (I) suivant la revendication 1 pour combattre des parasites.

**7.** Procédé de préparation de compositions pesticides, caractérisé en ce qu'on mélange des benzimidazoles substitués de formule (I) suivant la revendication 1 avec des diluants et/ou des agents tensio-actifs.